# EUROPEAN PATENT SPECIFICATION

(11) **EP 3 643 683 B1**
(45) Date of publication and mention of the grant of the patent: **23.10.2024**
(21) Application number: 18888697.2
(22) Date of filing: 07.12.2018
(51) Int. Cl.: C02F 1/44, F03G 7/00, H02S 10/10, H02S 40/42, H02S 40/44, C12M 1/00, H02N 11/00, H02S 10/30, C02F 103/08, C02F 1/14, C02F 1/469, F03G 6/00, H01L 31/048

(54) **SALINITY GRADIENT/SOLAR ENERGY HYBRID POWER GENERATION APPARATUS AND DESALINATION SYSTEM USING SAME**
SALINITÄTSGRADIENT/SOLARENERGIE-HYBRIDSTROMERZEUGUNGSVORRICHTUNG FÜR UND ENTSALZUNGSSYSTEM UNTER VERWENDUNG DAVON
APPAREIL DE PRODUCTION D'ÉNERGIE HYBRIDE À GRADIENT DE SALINITÉ/ÉNERGIE SOLAIRE ET SYSTÈME DE DESSALEMENT L'UTILISANT

(30) Priority: 11.12.2017 KR 20170169430; 03.08.2018 KR 20180090924; 01.10.2018 KR 20180117111
(43) Date of publication of application: 29.04.2020
(73) Proprietor: Korea Institute of Energy Research, Daejeon 34129 (KR)
(72) Inventor: JEONG, Nam Jo, Jeju-si, Jeju-do 63101 (KR); KIM, Han Ki, Jeju-si, Jeju-do 63226 (KR); HWANG, Kyo Sik, Jeju-si, Jeju-do 63326 (KR); NAM, Joo Youn, Jeju-si, Jeju-do 63229 (KR); YANG, Seung Cheol, Jeju-si, Jeju-do 63303 (KR); CHOI, Ji Yeon, Jeju-si, Jeju-do 63357 (KR); HAN, Ji Hyung, Jeju-si, Jeju-do 63303 (KR); JWA, Eun Jin, Jeju-si, Jeju-do 63336 (KR); PARK, Soon Chul, Jeju-si, Jeju-do 63357 (KR); SEO, Yong Seog, Daejeon 34120 (KR)
(74) Representative: Murgitroyd & Company
(86) International application number: PCT/KR2018/015332
(87) International publication number: WO 2019/117532

(56) References cited:
- EP-A1- 1 746 680
- WO-A1-2011/050473
- CN-A- 105 308 317
- JP-A- 2004 335 312
- JP-A- 2012 210 041
- KR-A- 20160 059 438
- KR-A- 20160 148 386
- KR-B1- 101 328 433

## Description

### [Technical Field]

The present invention relates to a salinity gradient and solar energy hybrid power generation apparatus using both salinity gradient power generation and solar energy power generation and a desalination system using the same, and more particularly, to a hybrid power generation apparatus which associates a salinity gradient and solar energy hybrid power generation apparatus with a desalination system.

### [Background Art]

Reverse electrodialysis (RED) refers to a technique that harvests, in the form of electrical energy, salinity gradient (salt gradient) energy generated in a process of mixing two fluids with different concentrations, for example, seawater and freshwater.

In more detail, RED is a system for generating power based on salinity gradient using seawater and freshwater. In the process, ions pass through ion exchange membranes (a cation exchange membrane and an anion exchange membrane) due to a concentration difference between salinity contained in seawater and salinity contained in freshwater. The ion migration causes an electric potential difference between electrodes (a positive electrode and a negative electrode) located at both ends of a stack in which a plurality of ion exchange membranes are alternately arranged. Electrical energy is generated through an oxidation-reduction (redox) reaction on the electrodes.

That is, RED is a power generation apparatus using a power generation scheme capable of directly converting, into electrical energy, chemical energy generated by ions that have been dissolved in seawater (saltwater) and that migrate to freshwater through an ion exchange membrane. The power generation scheme has little load variation and a utilization rate of 90% or more compared to conventional power generation schemes such as wind power generation and solar photovoltaic power generation.

Also, through a membrane installed between two fluids with different concentrations, for example, seawater and freshwater, freshwater which has a relatively low concentration flows toward seawater which has a relatively high concentration. Pressure retarded osmosis (PRO) is a process of generating electrical energy using fluid pressure energy that is generated along with an increase in flow toward the seawater.

In addition, solar thermal power generation is a scheme of heating water or air with solar thermal energy and using the heated water or air for heating or hot water, and solar photovoltaic power generation is a renewable energy scheme of converting solar energy into electrical energy using a solar cell panel.

Meanwhile, due to the increasing population and the desertification caused by global warming, water scarcity areas tend to be increasing. Therefore, in order to overcome the water scarcity, techniques to remove salinity from saltwater such as seawater have been developed.

However, despite the groundbreaking desalination idea, the desalination process has a higher energy cost than the general treatment of river water. In the case of a reverse osmosis process, which is a typical desalination process, 3-5 kWh/m³ of energy is used for freshwater production. Also, as a result of saltwater desalination, a large amount of high-concentrated saltwater is inevitably generated in addition to freshwater. Such concentrated saline wastewater has a salt concentration of about 7.0 wt%, which is twice as great as that of seawater, and thus may have a fatal effect on nearby marine ecosystems when discharged directly to sea. Therefore, the wastewater is classified as a marine pollutant and should be discharged while being lowered to a concentration lower than that of seawater.

Accordingly, there is a need to develop energy-efficient systems and processes that can lower the energy cost consumed in freshwater production while minimizing an environmental impact due to high-concentrated saline wastewater.

CN105308317, EP1746680, WO2011/050473, KR20160148386, KR20160059438, JP2012210041 may provide useful background to the present disclosure.

### [Disclosure]

### [Technical Problem]

The present invention is according to claim 1. The invention is directed to providing a salinity gradient and solar energy hybrid power generation apparatus capable of salinity gradient power generation and solar photovoltaic and solar thermal power generation, wherein the salinity gradient and solar energy hybrid power generation apparatus can efficiently increase electricity production by producing electrical energy using solar photovoltaic power generation and/or solar thermal power generation and by supplying hot water produced upon the production of the electrical energy to a reverse electrodialysis (RED) apparatus and/or a pressure retarded osmosis (PRO) apparatus.

Also, the present invention is directed to providing an energy-efficient freshwater system capable of minimizing environmental impact due to high concentration of waste saltwater.

Also, the present invention is directed to providing a process for compensating for energy used for desalination by implementing a salinity gradient and solar energy (e.g., sunlight and solar heat) hybrid power generation apparatus to maximize the amount of energy production in order to minimize the amount of energy consumed for desalination.

Also, the present invention is directed to providing an apparatus capable of reducing the amount of energy consumption and reusing concentrated wastewater by implementing a hybrid power generation system available to both of front and rear stages of a seawater desalination process.

Also, the present invention is directed to providing a technique to culture marine microalgae using diluted water discharged from hybrid power generation.

Also, the present invention is directed to providing an apparatus capable of extending the lifetime of a salinity gradient power generation system by improving the states of seawater and freshwater flowing into a salinity gradient power generation apparatus and also capable of improving the output power of a solar photovoltaic power generation apparatus and the performance of salinity gradient power generation by providing seawater and freshwater used for the salinity gradient power generation to a solar photovoltaic power generation system as cooling water.

### [Technical Solution]

According to the present invention, there is provided a salinity gradient and solar energy hybrid power generation apparatus as set out in the appended claims. The apparatus includes a solar photovoltaic power generation and/or solar thermal power generation unit supplied with first freshwater as cooling water; and a salinity gradient power generation unit supplied with first saltwater and the first freshwater raised in temperature due to heat exchange with the solar photovoltaic power generation and/or solar thermal power generation unit to generate electricity by a salinity gradient between the first freshwater and the first saltwater.

Also, the salinity gradient and solar energy hybrid power generation apparatus may further include a desalination unit supplied with electrical energy produced by the salinity gradient power generation unit.

Also, the salinity gradient and solar energy hybrid power generation apparatus may be disposed at a front or rear stage of the desalination unit.

Also, second saltwater having a lower concentration than the first saltwater may flow out of the salinity gradient and solar energy hybrid power generation apparatus and be supplied to and then desalinated by the desalination unit when the salinity gradient and solar energy hybrid power generation apparatus is disposed at the front stage of the desalination unit. The first saltwater supplied to the salinity gradient power generation unit may be discharged from the desalination unit when the salinity gradient and solar energy hybrid power generation apparatus is disposed at the rear stage of the desalination unit.

Also, the salinity gradient and solar energy hybrid power generation apparatus may further include at least one ion filter provided to filter out organic matter or a polyvalent ion contained in the supplied first saltwater and first freshwater.

Also, the salinity gradient and solar energy hybrid power generation apparatus may further include a pre-treatment unit provided to remove a floating substance contained in the first freshwater and the first saltwater before the first freshwater and the first saltwater are supplied to the solar photovoltaic power generation and/or solar thermal power generation unit and the salinity gradient power generation unit, respectively.

Also, the solar photovoltaic power generation and/or solar thermal power generation unit includes a solar cell panel having a first surface having a light receiving portion and a second surface opposite to the first surface. The salinity gradient power generation unit includes a housing having a first freshwater inflow port, a first freshwater outflow port, a first saltwater inflow port, and a first saltwater outflow port; an anode and a cathode disposed in the housing and spaced a predetermined distance from each other; and a plurality of ion exchange membranes arranged between the anode and the cathode to compart a first flow path through which the first freshwater flows and a second flow path through which the first saltwater flows. The housing has a first freshwater inflow portion configured to fluidly connect the first freshwater inflow port and the first flow path. The first freshwater inflow portion is provided to achieve heat exchange between the second surface of the solar cell panel and the influent first freshwater.

Also, the first freshwater inflow portion has at least one surface forming a space into which the first freshwater flows, the surface being the second surface of the solar cell panel.

Also, when the first freshwater flows into the first freshwater inflow portion, the first freshwater is brought into contact with the second surface of the solar cell panel.

Also, the salinity gradient power generation unit may further include a first inflow member provided to distribute the first freshwater to flow into a plurality of the first flow paths without the first freshwater flows into the second flow path.

Also, the first freshwater inflow portion may further include a first connection flow path configured to fluidly connect the first freshwater inflow portion and the first inflow member to deliver the first freshwater toward the first flow path.

Also, the plurality of ion exchange membranes may comprise a cation exchange membrane and an anion exchange membrane, and the cation exchange membrane and the anion exchange membrane are alternately placed.

Also, the salinity gradient and solar energy hybrid power generation apparatus may further include a marine microalgae culture device supplied with effluent water of the salinity gradient and solar energy hybrid power generation apparatus.

The desalination unit ay be a combination of a reverse osmosis (RO) apparatus and a pressure retarded osmosis (PRO) apparatus.

### [Advantageous Effects]

According to the present invention, it is possible to more efficiently produce energy through the salinity gradient and solar energy hybrid power generation apparatus.

Also, by using freshwater supplied to the salinity gradient power generation unit as cooling water for power generation using solar energy and also supplying the freshwater raised in temperature to a reverse electrodialysis (RED) apparatus, it is possible to achieve a synergy effect of improving both of the output power produced by the solar energy power generation apparatus and the output power produced by the salinity gradient power generation unit.

Also, through the hybrid power generation apparatus, it is possible to decrease the overall energy consumption of the desalination system, and it is also possible to reduce the salt concentration of concentrated saltwater generated by the desalination apparatus or minimize the production itself of the concentrated saltwater.

Also, generally, the solar photovoltaic power generation has a wide installation site of about 15,000 m² per MW while the salinity gradient power generation is evaluated as having an installation site of about 3,000 m² per MW. Therefore, through the configuration of the salinity gradient and solar energy hybrid power generation apparatus according to the present invention, it is possible to minimize the area of an available site required for the installation and maximize the amount of power generation.

Additionally, it is possible to utilize effluent water discharged through the hybrid power generation apparatus as an optimal source for culturing a variety of marine microalgae because the effluent water can be controlled in temperature and discharge concentration in the process of salinity gradient power generation.

### [Description of Drawings]

FIGS. 1 to 5 are schematic diagrams of desalination systems using salinity gradient and solar energy hybrid power generation apparatuses according to embodiments of the present invention.
FIGS. 6A to 6C show possible combinations of the hybrid power generation apparatuses applied to the above-described first to fifth embodiments.
FIGS. 7A and 7B are graphs showing cooling temperatures of a solar cell panel according to cooling methods and changes in temperature with time in a salinity gradient power generation inflow unit.
FIGS. 8 to 13 are diagrams showing a first embodiment of a rear-cooling-integrated hybrid power generation apparatus.
FIGS. 14 to 16 are diagrams showing a second embodiment of a rear-cooling-integrated hybrid power generation apparatus.
FIG. 17 is a graph showing power produced according to embodiments of the rear-cooling-integrated hybrid power generation apparatus.
FIGS. 18 and 19 are diagrams showing a third embodiment of a rear-cooling-integrated hybrid power generation apparatus.
FIGS. 20 to 22 are diagrams showing a fourth embodiment of a rear-cooling-integrated hybrid power generation apparatus.
FIGS. 23A to 23D are diagrams showing hybrid power generation systems provided by connecting a plurality of rear-cooling-integrated hybrid power generation apparatuses according to the first to fourth embodiments.
FIG. 24 is a diagram obtained by calculating an output power improvement rate of a hybrid power generation system according to a change in temperature on the basis of the embodiment of FIG. 17.
FIGS. 25A and 25B are diagrams showing hybrid power generation systems provided by connecting a plurality of salinity gradient power generation units to a solar cell panel.
FIG. 26 is a schematic diagram of a marine microalgae culture system using the hybrid power generation apparatus according to the second embodiment.
FIG. 27 is a block diagram showing a schematic structure of a power generation system using salinity gradient power generation according to an embodiment of the present invention.
FIG. 28 is a graph showing a contaminated total organic carbon (TOC) before and after freshwater passes through third and fourth ion filters.
FIG. 29 is a block diagram showing operation of an ion filter and a reverse electrodialysis salinity gradient power generator shown in FIG. 27.
FIG. 30 is a block diagram showing a schematic structure of a power generation system using salinity gradient power generation according to another embodiment of the present invention.

### [Best Modes]

Hereinafter, preferred embodiments of the present invention will be described in detail with reference to the accompanying drawings. Terms and words used in the specification and the claims shall not be interpreted as commonly-used dictionary meanings, but shall be interpreted to be relevant to the technical scope of the invention based on the fact that the inventor may properly define the concept of the terms to explain the invention in best ways.

In addition, irrespective of reference numerals, the same or corresponding components will be given the same or similar reference numerals, and a duplicate description thereof will be omitted. For the convenience of description, the size and shape of each component shown may be exaggerated or reduced.

Therefore, the embodiments described herein and the configurations illustrated in the drawings are for illustrative purposes only and are not intended to represent the entire technical scope of the invention, and thus it should be understood that there may be various available equivalents and modifications at the time of filing this application.

First, in this document, elements not mentioned separately in connection with a solar cell panel and a saline gradient power generation apparatus (a reverse electrodialysis power generation apparatus) may be appropriately selected and used by those skilled in the art according to the purpose and conditions of use if they have been conventionally used.

Also, throughout this document, the term "connection," "installation," or "mounting" refers to fixed connection using bolts and nuts. In this case, a connection member other than bolts and nuts may be appropriately selected and used by those skilled in the art.

Hereinafter, a salinity gradient and solar energy hybrid power generation apparatus and a desalination system using the same according to exemplary embodiments of the present invention will be described in detail.

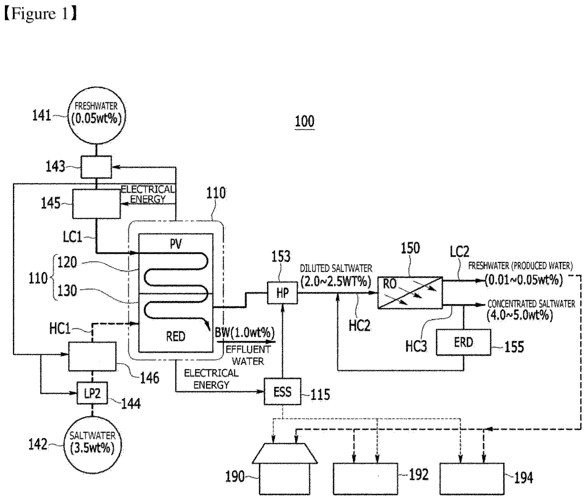

FIGS. 1 to 5 are schematic diagrams of desalination systems using salinity gradient and solar energy hybrid power generation apparatuses (hereinafter referred to as desalination systems) according to embodiments of the present invention.

A desalination system according to an embodiment of the present invention includes a hybrid power generation apparatus including a solar photovoltaic power generation and/or solar thermal power generation unit supplied with first freshwater as cooling water and a salinity gradient power generation unit supplied with first saltwater and the first freshwater raised in temperature by cooling the solar photovoltaic power generation and/or solar thermal power generation unit; and a desalination apparatus supplied with electrical energy generated by the hybrid power generation apparatus.

Also, the hybrid power generation apparatus is installed at a front stage of the desalination apparatus. Second saltwater that flows out of the hybrid power generation apparatus and has a lower concentration than the first saltwater is supplied to the desalination apparatus. The desalination apparatus produces freshwater through desalination of the second saltwater.

Also, the hybrid power generation apparatus is installed at a rear stage of the desalination apparatus, and the first saltwater is saltwater flowing out of the desalination apparatus.

Also, the second saltwater that flows out of the salinity gradient power generation unit and that has a lower concentration than the first saltwater is resupplied to the seawater desalination apparatus.

Also, the hybrid power generation apparatus is installed at the front stage of the desalination apparatus. Second saltwater that flows out of the hybrid power generation apparatus and has a lower concentration than the first saltwater is supplied to the desalination apparatus. The desalination apparatus produces freshwater through desalination of the second saltwater. The hybrid power generation apparatus further includes an additional salinity gradient power generation unit supplied with brackish water flowing out of the salinity gradient power generation unit and third saltwater that is generated in the desalination apparatus and that has a higher concentration than the second saltwater.

Also, the hybrid power generation apparatus is provided at both of the front stage and the rear stage of the desalination apparatus.

Also, the hybrid power generation apparatus is provided at the front stage of the desalination apparatus, and the desalination apparatus includes a combination of a reverse osmosis (RO) apparatus and a pressure retarded osmosis (PRO) apparatus.

Also, the hybrid power generation apparatus includes the solar photovoltaic power generation and/or solar thermal power generation unit and the salinity gradient power generation unit formed in a rear-cooling-integrated manner.

Also, the hybrid power generation further includes a marine microalgae culture device supplied with effluent water of the hybrid power generation apparatus.

First, the desalination system according to embodiments of the present invention generates energy using the salinity gradient and solar energy hybrid power generation apparatus including the solar photovoltaic power generation and/or solar thermal power generation unit and the salinity gradient power generation unit (hereinafter referred to as the hybrid power generation apparatus) and also generates freshwater by using diluted saltwater flowing out of the hybrid power generation apparatus as a source of the desalination apparatus and the marine microalgae culture device.

In particular, since freshwater supplied to the solar photovoltaic power generation and/or solar thermal power generation unit is used as cooling water of the solar photovoltaic power generation and/or solar thermal power generation unit, and also the freshwater raised in temperature after used as the cooling water of the solar photovoltaic power generation and/or solar thermal power generation unit is supplied to the salinity gradient power generation unit. Thus, it is possible to exhibit a synergy effect in that both output power produced by the solar photovoltaic power generation and/or solar thermal power generation unit and output power produced by the salinity gradient power generation unit are improved.

Referring to FIGS. 1 to 5, desalination systems 100, 100a, 100b, 100c, and 100d of the present invention each include a hybrid power generation apparatus 110 including a solar photovoltaic power generation and/or solar thermal power generation unit 120 and a salinity gradient power generation unit 130 and a desalination unit 150.

In detail, FIG. 1 shows a schematic diagram of a desalination system 100 using hybrid power generation according to a first embodiment of the present invention.

The desalination system 100 according to the first embodiment of the present invention comprises a hybrid power generation apparatus 110 and a desalination apparatus 150. The hybrid power generation apparatus 110 includes a solar photovoltaic power generation and/or solar thermal power generation unit 120 and a salinity gradient power generation unit 130.

First freshwater LC1 is supplied to the solar photovoltaic power generation and/or solar thermal power generation unit 120 through a first freshwater supply unit (a first supply unit) 141. The first freshwater LC1 is a low concentration solution (0.005 to 1.0 wt%) and may include at least one selected from among general freshwater, groundwater, rainwater, sewage effluent water, general agricultural water, washing water, cooling water, and mixtures thereof. However, the present invention is not limited thereto.

The first freshwater LC1 is supplied to the solar photovoltaic power generation and/or solar thermal power generation unit 120 as cooling water. The solar photovoltaic power generation and/or solar thermal power generation unit 120 is raised in temperature by sunlight or solar heat received from a light-receiving unit and thus is cooled by the supplied first freshwater LC1. Accordingly, the output power performance of the solar photovoltaic power generation and/or solar thermal power generation unit 120 is improved compared to the case where the first freshwater LC1 is not supplied.

Meanwhile, the first freshwater LC1 having passed through the solar photovoltaic power generation and/or solar thermal power generation unit 120 is raised in temperature by heat received from the solar photovoltaic power generation and/or solar thermal power generation unit 120. The first freshwater LC1 raised in temperature is supplied to the salinity gradient power generation unit 30, and first saltwater HC1 is also supplied to the salinity gradient power generation unit 30 through a first saltwater supply unit (a second supply unit) 142. The first saltwater HC1 is a high concentration solution (1.0 to 50.0 wt%) and may include at least one selected from among saltwater (7.0 wt% or higher), seawater (3.0 to 3.5 wt%), fertilizer solution (1.0 to 50.0 wt%), carbon dioxide absorption solution (1.3 to 50.0 wt%), salt solution obtained through fuel pretreatment for various power plants (3.5 to 50.0 wt%), and mixtures thereof. However, the present invention is not limited thereto.

Here, solar photovoltaic power generation refers to a scheme of converting solar energy into electrical energy using a solar cell panel, and solar thermal power generation refers to a scheme of heating water or air with solar thermal energy and using the heated water or air for heating or hot water. The solar photovoltaic power generation and/or solar thermal power generation unit 120 may be provided in either or a combination of the two.

The salinity gradient power generation unit 130 produces electricity using a salinity gradient between the supplied first freshwater LC1 and first saltwater HC1. A PRO apparatus or a reverse electrodialysis (RED) apparatus may be used as the salinity gradient power generation unit 130. In FIG. 1, an RED apparatus is illustrated as the salinity gradient power generation unit 130.

When freshwater LC and saltwater HC intersect each other with an osmosis membrane provided as a boundary, water at the freshwater side moves to the saltwater side through the osmosis membrane, which causes an increase in pressure. The PRO apparatus is a power generation apparatus capable of utilizing the pressure increase as a driving source of a power generation turbine. As an example, a PRO apparatus disclosed in KR7458429, which was applied by the applicant and registered, may be used as the PRO apparatus. However, various PRO apparatuses are available.

In the RED, ions pass through ion exchange membranes (a cation exchange membrane and an anion exchange membrane) due to a concentration difference between freshwater LC and saltwater HC. The ion migration causes an electric potential difference between both ends of a cell stack in which a cation exchange membrane and an anion exchange membrane are alternately placed. Electricity is generated due to a flow of electrons through a redox reaction on the electrodes (a positive electrode and a negative electrode).

The RED is more suitable as the salinity gradient power generation unit 130 than the PRO in that it is possible to supply a purer solution as diluted saltwater HC2 supplied to the desalination unit 150, reduce the throughput of the desalination unit 150, and consequently reduce energy consumption.

The first freshwater LC1 supplied to the salinity gradient power generation unit 130 is raised in temperature by passing through the solar photovoltaic power generation and/or solar thermal power generation unit 120. As a result, the moving speed of ionic substances in the first saltwater HC1 is increased, thereby increasing power production.

Meanwhile, a first pressure adjustment unit 143 and a first pre-treatment unit 145 may be installed at the first supply unit (the first freshwater supply unit) 141 while a second pressure adjustment unit 144 and a second pre-treatment unit 146 may be installed at the second supply unit (the first saltwater supply unit) 142. The first pre-treatment unit 145 may be microfiltration (MF) or dissolved air flotation (DAF). The second pre-treatment unit 146 may be ultrafiltration (UF) or dissolved air flotation (DAF). The first saltwater HC1 may be supplied after microorganisms, suspended substances, and the like contained in the first saltwater HC1 are removed through the first and second pre-treatment units 145 and 146.

From the salinity gradient power generation unit 130, effluent water (about 1.0 wt%) BW having a higher concentration than the first freshwater LC1 (with respect to 0.01 wt%) is discharged, and also the second saltwater HC2 having a lower concentration than the first saltwater HC1 is discharged. The second saltwater HC2 is supplied to the desalination unit 150.

The desalination unit 150 may desalinate the supplied second saltwater HC2 to generate third saltwater HC3 and also second freshwater LC2. The second freshwater LC2 is freshwater having a concentration lower than or equal to that of the first freshwater LC1 and may be post-treated to meet desired water quality standards if necessary. The third saltwater HC3 may have a higher concentration than the second saltwater HC2. For example, when the concentration of the first saltwater HC1 is 3.5 wt%, the concentration of the second saltwater HC2 may be from about 2.0 to 2.5 wt%, and the concentration of the third saltwater HC3 may be from 4.0 to 5.0 wt%.

The desalination unit 150 may use Multi-Effect Distillation (MED), Multiple-Stage Flash Distillation (MSF), Mechanical Vapor Compression Distillation (MVC), Thermo Vapor Compressor Distillation (TVC), Electrodialysis (ED), Capacitive Deionization (CDI), Reverse Osmosis (RO), Forward Osmosis (FO), or the like. Depending on the installation environments and conditions, a saltwater desalination process may be used in a hybrid manner, i.e., in a combination of one or more of the above schemes. In FIG. 1, the RO is illustrated as the desalination unit 150.

The desalination unit 150 may include a plurality of desalination units 150 combined either in series or in parallel depending on the concentration the supplied second saltwater HC2 and the concentration of the discharged third saltwater HC3.

The desalination unit 150 may further include an energy recovery apparatus 155. The energy recovery apparatus 155 is an apparatus for recovering a portion of energy used for desalination. For example, when the desalination unit 150 is an RO apparatus, the energy recovery apparatus 155 may be an apparatus for recovering energy used for desalination as pressure.

A third pressure adjustment unit 153 may be arranged at a front stage of the desalination unit 150. The third pressure adjustment unit 153 may be a highpressure pump for providing a driving force needed to overcome the high osmotic pressure of the second saltwater HC2 to produce the second freshwater LC2.

A portion of electrical energy produced by the hybrid power generation apparatus 110 may be supplied to the first pressure adjustment unit 143, the second pressure adjustment unit 144, the third pressure adjustment unit 153, the first pre-treatment unit 145, and the second pre-treatment unit 146. Also, the electrical energy produced by the hybrid power generation apparatus 110 may be stored in an energy storage system ESS 115 and then supplied.

Also, the concentration of the third saltwater HC3 that is finally discharged by the desalination system 100a using the hybrid power generation according to an embodiment is from 4 to 5 wt%, which is lower than that of the saltwater discharged when only a desalination unit is provided without hybrid power generation, i.e., 7.0 wt%.

The electrical energy and the freshwater (produced water) produced by the desalination system 100a may be supplied to a smart house 190, a smart factory facility 192, a smart farm 194, etc., which require energy and/or freshwater.

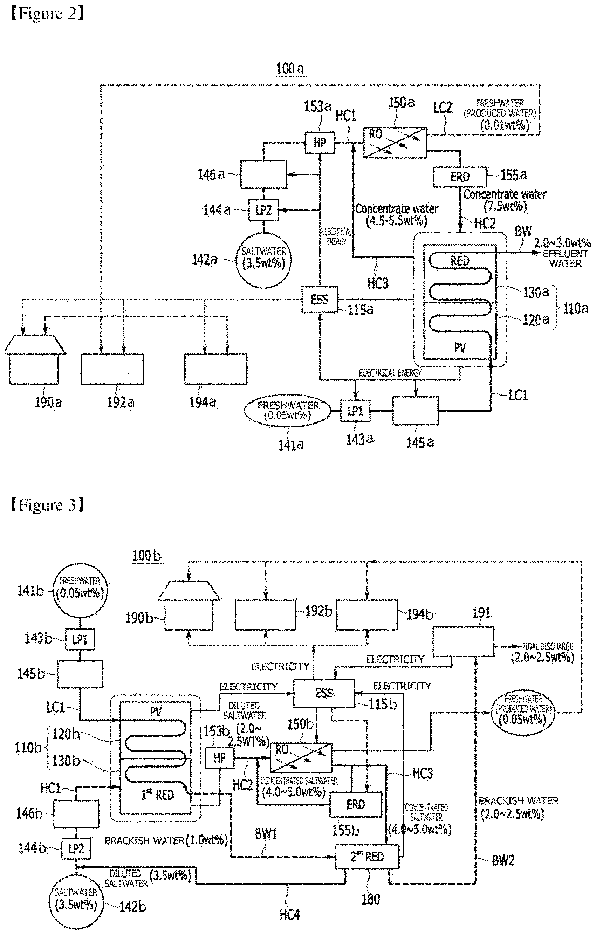

Meanwhile, FIG. 2 is a schematic diagram of a desalination system 100a using hybrid power generation according to a second embodiment of the present invention.

Referring to FIG. 2, the desalination system 100a using hybrid power generation according to the second embodiment of the present invention comprises a hybrid power generation apparatus 110a at a rear stage of a desalination unit 150a. Like the embodiment illustrated in FIG. 1, the hybrid power generation apparatus 110a comprises a solar photovoltaic power generation and/or solar thermal power generation unit 120a and a salinity gradient power generation unit 130a.

First freshwater LC1 is supplied to the solar photovoltaic power generation and/or solar thermal power generation unit 120a through a first freshwater supply unit 141a as cooling water, and thus it is possible to increase the power production of the solar photovoltaic power generation and/or solar thermal power generation unit 120a.

When first saltwater HC1 is supplied to the desalination unit 150a through a first saltwater supply unit 142a, the desalination unit 150a produces second freshwater LC2 and second saltwater HC2, which is concentrated saltwater. The second freshwater LC2 is freshwater having a concentration lower than or equal to that of the first freshwater LC1 and may be post-treated to meet desired water quality standards if necessary. The second saltwater HC2 may have a higher concentration than the second saltwater HC2. For example, when the concentration of the first saltwater HC1 is about 3.5 wt%, the concentration of the second saltwater HC2 may be about 7.5 wt%.

Like the first embodiment, the desalination unit 150a may further include an energy recovery apparatus 155a. When the desalination unit 150a is an RO apparatus, the energy recovery apparatus 155a may be an apparatus for recovering energy used for desalination as pressure.

When the second saltwater HC2 flowing out of the desalination unit 150a and the first freshwater LC1 raised in temperature by being used in the solar photovoltaic power generation and/or solar thermal power generation unit 120a as cooling water are supplied to the salinity gradient power generation unit 130a, electrical energy is generated due to the increased moving speed and the concentration difference between the second saltwater HC2 and the first freshwater LC1 raised in temperature.

The electrical energy generated in the hybrid power generation apparatus 110a may be used to provide a driving force to a first pressure adjustment unit 143a and a first pre-treatment unit 145a arranged at a front stage of the solar photovoltaic power generation and/or solar thermal power generation unit 120a and a second pressure adjustment unit 144a, a second pre-treatment unit 146a, and a third pressure adjustment unit 153a installed at a front stage of the desalination unit 150a.

Third saltwater HC3 flowing out of the salinity gradient power generation unit 130a may have a lower salt concentration than the second saltwater HC2 supplied to the salinity gradient power generation unit 230. For example, the salt concentration of the third saltwater HC3 may be from about 5.0 to 5.6 wt%. The third saltwater HC3 may not flow to the outside, but may be supplied to the desalination unit 150a together with the first saltwater HC1.

Accordingly, in the desalination system 100a according to the second embodiment, concentrated saltwater generated in the desalination unit 150a is not exported to the outside, but is used as a source of the salinity gradient power generation unit 130a to generate saltwater with a decreased salt concentration and then re-supply the saltwater to the desalination unit 150a for the purpose of recycling. Thus, it is possible to solve an environmental problem caused by a high concentration of saltwater generated during a conventional desalination process.

Meanwhile, FIG. 3 is a schematic diagram of a desalination system 100b according to a third embodiment of the present invention.

Referring to FIG. 3, the desalination system 100b according to the third embodiment further comprises a second salinity gradient power generation unit 180 at a rear stage of a desalination unit 150b unlike the first embodiment.

For convenience of description, a description of the same elements as those of the first embodiment will be omitted. When third saltwater HC3 flowing out of the desalination unit 150b and first brackish water BW1 flowing out of a first salinity gradient power generation unit 130b are supplied to the second salinity gradient power generation unit 180, additional energy is generated, and also second brackish water BW2 and fourth saltwater HC4 having a lower concentration than the third saltwater HC3 are discharged. The fourth saltwater HC4 may be reused as first saltwater HC1, which is a source of the first salinity gradient power generation unit 130b, and the second brackish water BW2 is supplied for small hydro power generation or tidal current power generation 191. Finally, saltwater having a concentration of about 2.0 to 2.5 wt% is discharged, and the salt concentration of the saltwater is lower than that (7.0 wt%) of saltwater discharged when only a desalination unit is used by a factor of about 1/3 or less, and thus it is possible to reduce the impact on the environment.

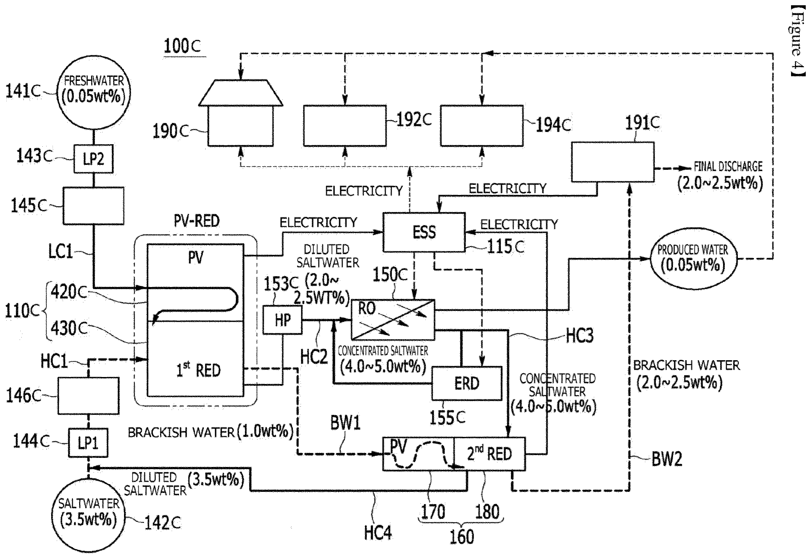

FIG. 4 is a schematic diagram of a desalination system 100c according to a fourth embodiment of the present invention.

Referring to FIG. 4, the desalination system 100c according to the fourth embodiment further comprises a second hybrid power generation apparatus 160 at a rear stage of a desalination unit 150c unlike the first embodiment. For convenience of description, a description of the same elements as those of the first embodiment will be omitted.

The second hybrid power generation apparatus 160 includes a second solar photovoltaic power generation and/or solar thermal power generation unit 170 and a second salinity gradient power generation unit 180. Brackish water BW1 flowing out of a first salinity gradient power generation unit 130c of a first hybrid power generation apparatus 110c is supplied to the second solar photovoltaic power generation and/or solar thermal power generation unit 170 of the second hybrid power generation apparatus 160 as cooling water. Then, when the brackish water BW1 raised in temperature after a cooling function is performed is supplied to the second salinity gradient power generation unit 180, the second salinity gradient power generation unit 180 discharges second brackish water BW2 and fourth saltwater HC4 having a lower concentration than third saltwater HC3. The fourth saltwater HC4 may be reused as first saltwater HC1, which is a source of the first salinity gradient power generation unit 130c, and second brackish water BW2 is supplied for small hydro power generation or tidal current power generation 191c. Finally, saltwater having a concentration of about 2.0 to 2.5 wt% is discharged, and the salt concentration of the saltwater is lower than that (7.0 wt%) of saltwater discharged when only a desalination unit is used by a factor of about 1/3 or less, and thus it is possible to reduce the impact on the environment.

Also, additional energy may be generated through the second hybrid power generation apparatus 160 and the small hydro power generation or tidal current power generation 191c and the electrical energy and fresh water (produced water) may be supplied to a smart house 190, a smart factory facility 192, a smart farm 194, etc., which require energy and/or freshwater.

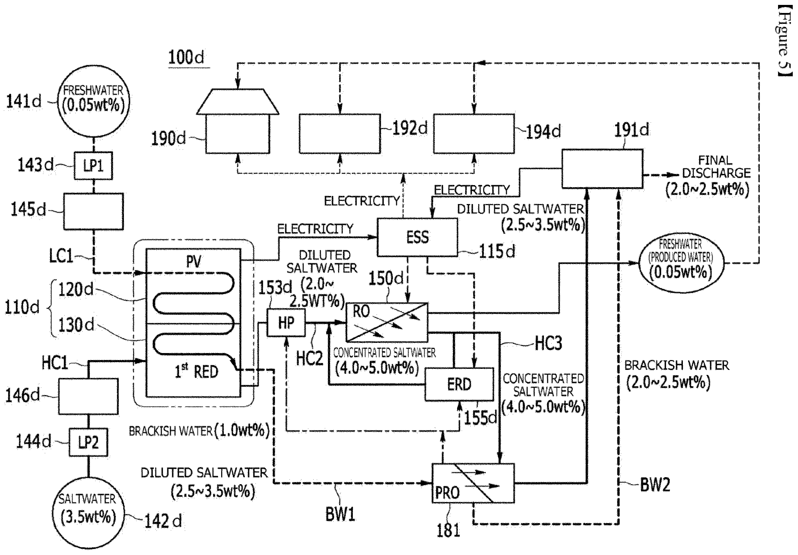

FIG. 5 is a schematic diagram of a desalination system 100d according to a fifth embodiment of the present invention.

Referring to FIG. 5, the desalination system 100d according to the fifth embodiment further comprises a PRO apparatus 181 at a rear stage of a desalination unit 150d unlike the first embodiment. When first brackish water BW1 flowing out of a salinity gradient power generation unit 130d of the hybrid power generation apparatus 110d and third saltwater HC3 flowing out of the saltwater desalination unit 150d composed of RO are supplied, electrical energy is produced due to a difference in osmotic pressure between the first brackish water BW1 and the third saltwater HC3. Also, second brackish water BW2 and fourth saltwater HC4 flowing out of the PRO apparatus 181 are supplied to a small hydro power generation or tidal current power generation apparatus 191d to generate additional energy.

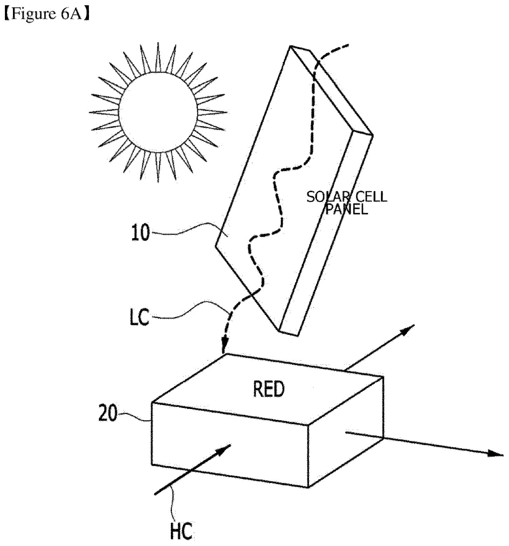

FIGS. 6A to 6C show possible combinations of the hybrid power generation apparatuses applied to the above-described first to fifth embodiments.

In FIGS. 6A to 6C, a solar cell panel 10 will be described as an example of solar photovoltaic power generation and/or solar thermal power generation units 120, 120a, 120b, 120c, and 120d.

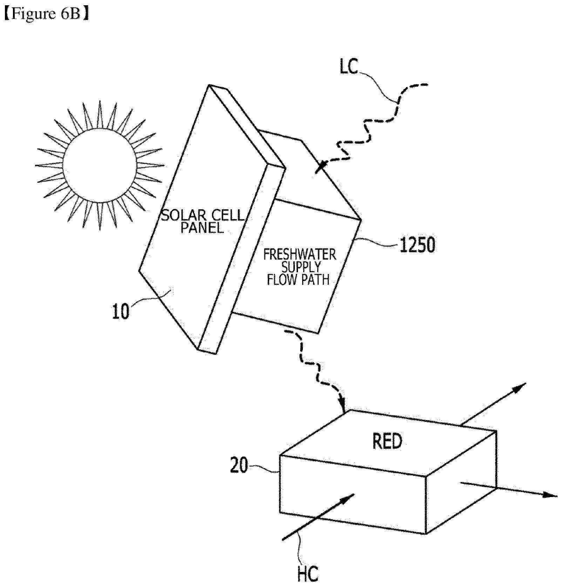

FIG. 6A shows a front-cooling-separation type in which freshwater LC is supplied to the front side of a solar cell panel 10 and then supplied to a salinity gradient power generation unit 20. FIG. 6B shows a rear-cooling-separation type in which freshwater LC is supplied through a freshwater supply flow path 1250 equipped in the rear side of a solar cell panel 10 and then supplied to a salinity gradient power generation unit 20. FIG. 7C shows a rear-cooling-integration type in which a solar cell panel 10 and a salinity gradient power generation unit 20 are formed in an integrated and a freshwater supply flow path 1250 is provided between the solar cell panel 10 and the salinity gradient power generation unit 20.

The front-cooling-separation type shown in FIG. 6A has excellent cooling performance but may have limitations on a cooling time because it should not interfere with collection of sunlight. Partial heat loss may occur before freshwater LC flows into the salinity gradient power generation unit 20. The rear-cooling-separation type shown in FIG. 6B may have cooling performance varying depending on a cooling channel material and a flow path configuration scheme, but have no limitation on a cooling time. However, partial heat loss may occur before freshwater LC flows into the salinity gradient power generation unit 20. The rear-cooling-integration type shown in FIG. 7C has excellent cooling performance and no limitation on a cooling time, and thus it is possible to minimize heat loss before flowing into the salinity gradient power generation unit 20 and miniaturize the overall size of the hybrid power generation apparatus. In detail, generally, the solar photovoltaic power generation has a wide installation site of about 15,000 m² per MW while the salinity gradient power generation is evaluated as having an installation site of about 3,000 m² or less per MW. Therefore, when the salinity gradient power generation and the solar photovoltaic power generation are configured in an integrated manner, it is possible to minimize the area of an available site required for the installation and maximize the amount of power generation.

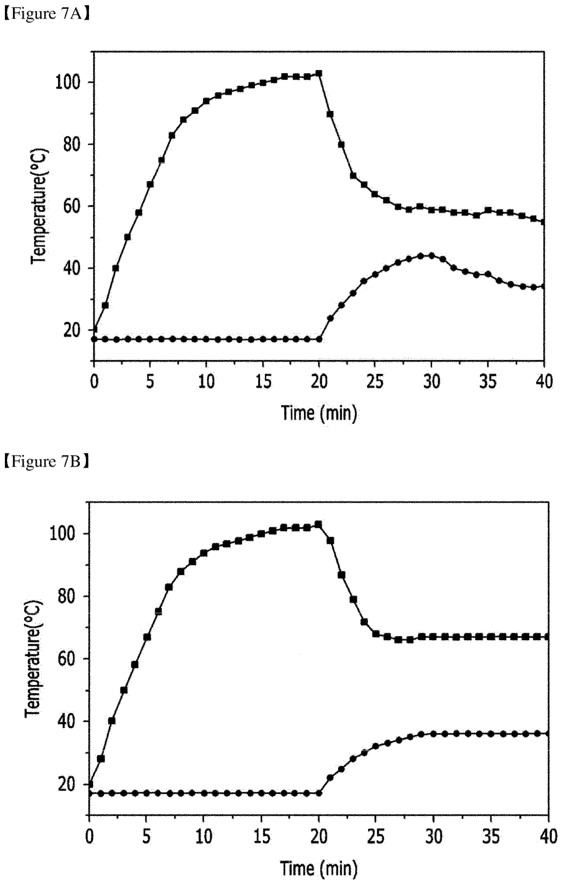

FIGS. 7A and 7B are graphs showing cooling temperatures of a solar cell panel according to cooling methods and changes in temperature with time in a salinity gradient power generation inflow unit. FIG. 7A shows a change in temperature with time when cooling water is supplied to the front side of the solar cell panel 10 and is used as a freshwater source of the salinity gradient power generation (RED).

The cooling scheme of FIG. 7A is front cooling where a helium bulb was used as artificial sunlight, a solar photovoltaic panel was 40W, a RED stack was 10W, a freshwater flow rate for cooling water was 1 L/min, a freshwater concentration was 0.01 wt%, a saltwater flow rate was 1 L/min, and a saltwater concentration was 3.0 wt%.

Referring to FIG. 7A, the temperature of the solar cell panel 10 rises up to about 100 degrees Celsius (a graph displayed using a square). In this case, when cooling water of 17°C was supplied to the front side of the solar cell panel 10, the temperature of the solar cell panel 10 dropped to about 56°C, and the temperature of cooling water supplied to a freshwater entrance of salinity gradient power generation 20 rose up to 45°C (a graph displayed using a circle). However, with the passage of time, the temperature of the cooling water supplied to the freshwater entrance of the salinity gradient power generation 20 dropped to about 34°C.

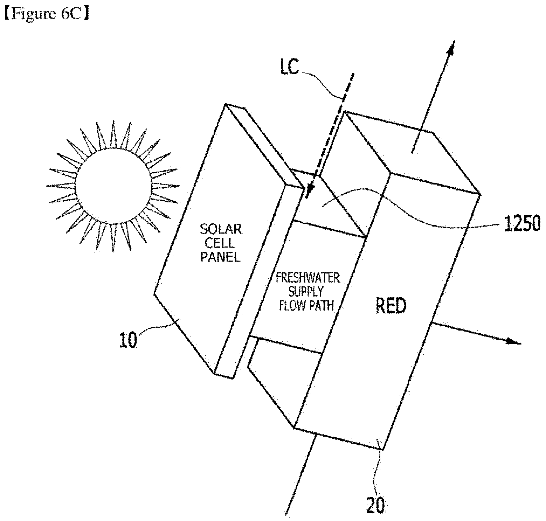

FIG. 7B shows a result of the rear-cooling schemes shown in FIGS. 6B and 6C. For the purpose of cooling, a flow of cooling water used a flow path structure patterned on the rear side.

The cooling scheme of FIG. 7B is rear cooling where a helium bulb was used as artificial sunlight, a solar photovoltaic panel was 40W, a RED stack was 10W, a freshwater flow rate for cooling water was 1 L/min, a freshwater concentration was 0.01 wt%, a saltwater flow rate was 1 L/min, and a saltwater concentration was 3.0 wt%.

In this case, when cooling water of 17°C was supplied to the front side, the temperature of the solar cell panel 10 dropped to about 67°C (a graph displayed using a square), and the temperature of cooling water supplied to a freshwater entrance of salinity gradient power generation rose up to 36°C (a graph displayed using a circle). It can be seen that the temperature of cooling water remained constant thereafter. As shown in FIGS. 7A and 7B, it is determined that the rear cooling is more advantageous in order to increase the output power of the solar cell panel 10 and stabilize the output power of the salinity gradient power generation 20 without load variation. The front cooling could aim at increasing the output power of the solar photovoltaic panel due to an instantaneous cooling effect. Therefore, it is determined that it will be necessary to mainly perform the rear cooling and intermittently perform the front cooling in order to increase the output power of the hybrid power generation and stabilize the power generation.

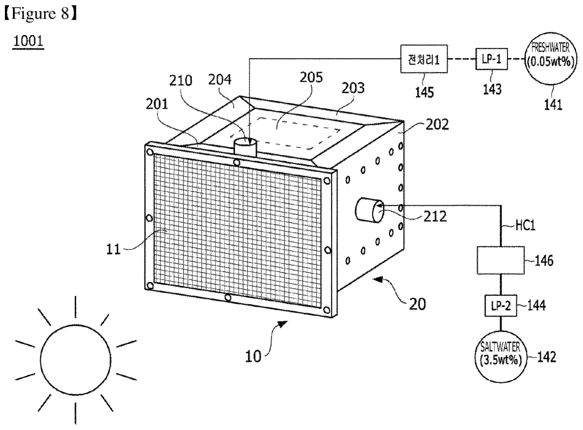

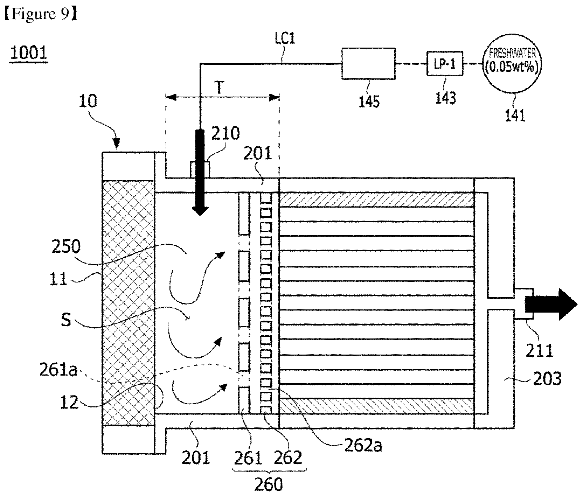

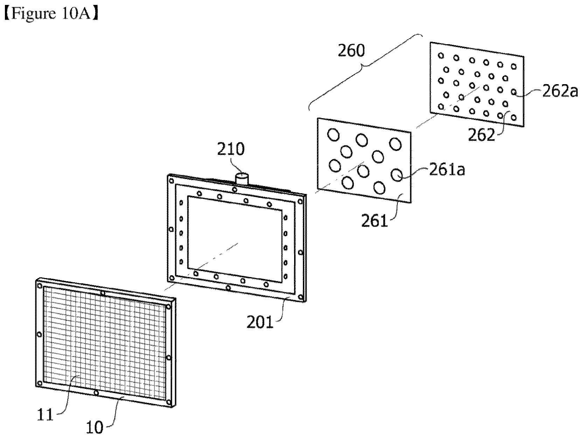

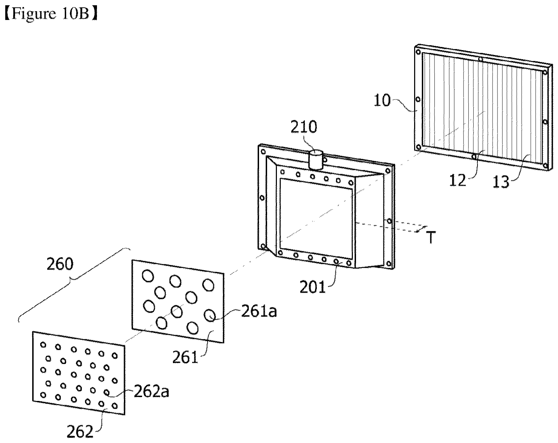

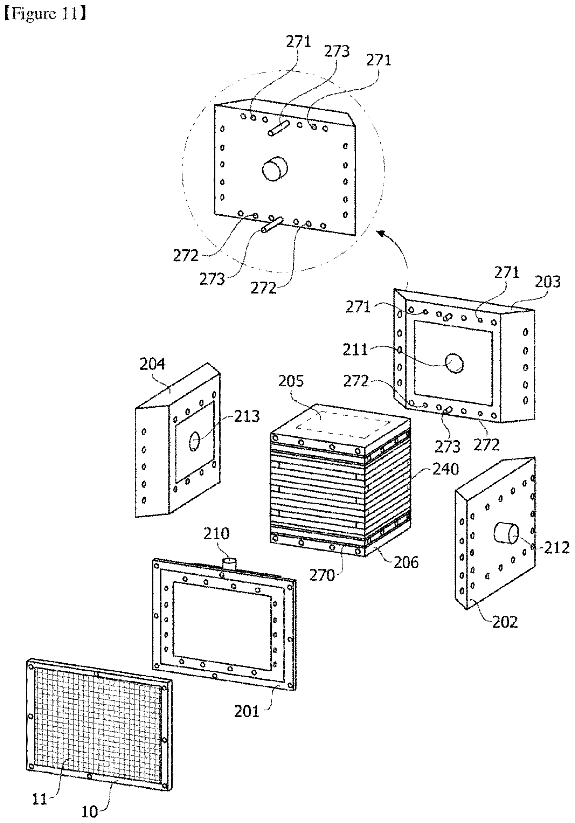

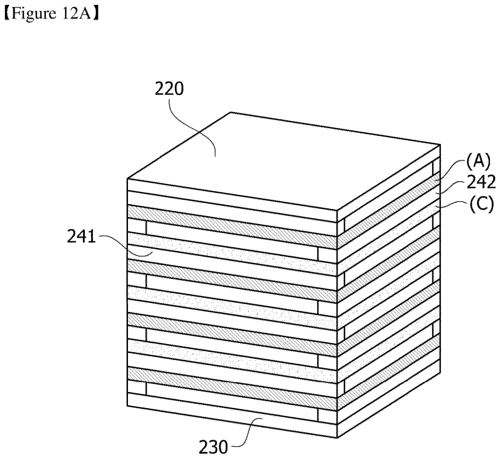

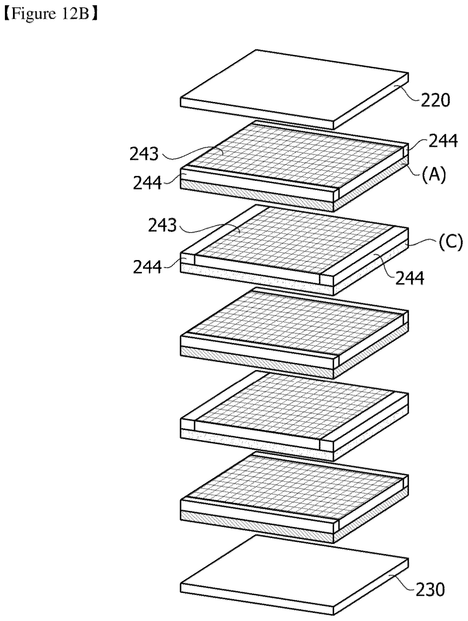

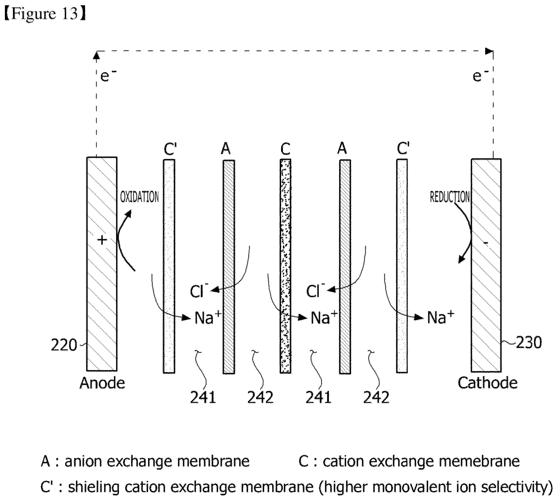

FIGS. 8 to 13 are diagrams showing a first embodiment of the rear-cooling-integrated hybrid power generation apparatus 110. FIGS. 8 and 9 are a perspective view and a side view of the first embodiment of the rear-cooling-integrated hybrid power generation apparatus. FIG. 10 is an exploded perspective view and a rear view illustrating a freshwater inflow portion (a first freshwater inflow portion) and a first inflow member of the first embodiment of the rear-cooling-integrated hybrid power generation apparatus. FIG. 11 is an exploded perspective view of the first embodiment of the rear-cooling-integrated hybrid power generation apparatus. FIGS. 12 and 13 are a perspective view and a schematic view illustrating a salinity gradient power generation unit.

As shown in FIGS. 8 to 13, a first embodiment 1001 of the rear-cooling-integrated hybrid power generation apparatus includes a solar cell panel 10 and a salinity gradient power generation unit 20.

The solar cell panel 10 includes a first surface 11 having a light-receiving portion and a second surface 12 opposite to the first surface 11. Here, the light receiving portion of the first surface 11 refers to an area where the solar cell panel can receive light and produce electricity.

In particular, referring to FIG. 10B, at least a portion of the second surface 12 may additionally include a heat dissipation member 13.

The heat dissipation member 13 may be formed in a structure capable of widening a heat dissipation area in order to increase heat dissipation efficiency. As an example, various structures such as a fin or heat pipe structure formed to protrude from the second surface 12 are available if they can widen a heat exchange area.

Also, the salinity gradient power generation unit 20 may receive first freshwater LC1 (hereinafter referred to as freshwater) and first saltwater HC1 (hereinafter referred to as saltwater) and produce electricity through salinity gradient power generation.

Here, the freshwater is a low concentration solution (0.005 to 1.0 wt%) and may include at least one selected from among general freshwater, groundwater, rainwater, sewage effluent water, general agricultural water, washing water, cooling water, and mixtures thereof. However, the present invention is not limited thereto.

In addition, the saltwater is a high concentration solution (1.0 to 50.0 wt%) and may include at least one selected from among saltwater (7.0 wt% or higher), seawater (3.5 wt%), fertilizer solution (1.0 to 50.0 wt%), carbon dioxide absorption solution (1.3 to 50.0 wt%), and mixtures thereof. However, the present invention is not limited thereto.

Here, the present invention may include a first supply unit (a first freshwater supply unit) 141 and a second supply unit (a first saltwater supply unit) 142 for supplying the freshwater LC1 and the saltwater HC1 to a freshwater (LC1) inflow port and a saltwater (HC1) inflow port, respectively.

In more detail, referring to FIGS. 11 to 13, the salinity gradient power generation unit 20 includes a housing 200 having a freshwater inflow port 210, a freshwater outflow port 211, a saltwater inflow port 212, and a saltwater outflow port 213.

The housing 200 includes an anode 220 and a cathode 230 disposed therein and spaced a predetermined distance from each other and a plurality of ion exchange membranes 240 placed between the anode 220 and the cathode 230.

The plurality of ion exchange membranes 240 may be arranged between the anode 220 and the cathode 230 to compart a first flow path 241 through which the freshwater LC1 flows and a second flow path 242 through which the saltwater HC1 flows.

Here, the plurality of ion exchange membranes 240 include a cation exchange membrane C and anion exchange membrane A, which may be alternately placed.

Accordingly, a plurality of first flow paths 241 and a plurality of second flow paths 242 may be formed in the housing 200 where the plurality of ion exchange membranes are arranged.

In more detail, the housing 200 of the salinity gradient power generation unit 20 according to the first embodiment of the rear-cooling-integrated hybrid power generation apparatus of the present invention includes first to fourth frames 201, 202, 203, and 204 where the freshwater inflow port 210, the saltwater inflow port 212, the freshwater outflow port 211, and the saltwater outflow port 213 are provided, respectively.

Also, the housing 200 includes fifth and sixth frames 205 and 206 provided to surround the anode 220 and the cathode 230, respectively.

Referring to FIG. 11, as an example, the housing 200 may be formed in a hexahedron shape. The first to fourth frames 201, 202, 203, and 204 may form the side surfaces of the housing 200, and the fifth and sixth frames 205 and 206 may form the upper and lower surfaces of the housing 200.

In more detail, the first frame 201 may include the freshwater inflow port 210. Also, the second frame 202 may include the saltwater inflow port 212. Also, the third frame 203 may include the freshwater outflow port 211. Also, the fourth frame 204 may include the saltwater outflow port 213. Here, the housing 200 may be formed by connecting the first to sixth frames to one another.

Also, each of the fifth and sixth frames may additionally include a sealing gasket 270 provided to prevent the freshwater LC1 and the saltwater HC1 flowing through the first and second flow paths 241 and 242 provided between the fifth and sixth frames 205 and 206 from flowing to the outside.

Also, the third frame 203 may additionally include an electrolyte inflow port 271 and an electrolyte outflow port 272 for supplying and discharging a solution, i.e., electrolyte to the anode 220 and the cathode 230.

Here, an electrolyte supply source (not shown) for supplying the electrolyte may be additionally included.

Also, the third frame 203 may additionally include an electrode pole 273 as a collector of electricity produced in the anode 220 and the cathode 230.

Accordingly, the electrode pole 273 may be provided at upper and lower ends of the third frame 203 and connected to the anode 220 and the cathode 230.

In particular, the electrode pole 273 may be formed to protrude to the outside of the third frame 203 and thus supply the produced electricity to the outside.

Meanwhile, referring to FIG. 9, the housing 200 includes a freshwater inflow portion 250 configured to fluidly connect the freshwater inflow port 210 and the first flow path 241.

Also, the freshwater inflow portion 250 may be provided to achieve heat exchange between the second surface 12 of the solar cell panel and the influent freshwater LC1.

In more detail, the freshwater inflow portion 250 of the present invention may have at least one surface forming a space S into which the freshwater LC1 flows, wherein the surface is the second surface 12 of the solar cell panel 10.

The first frame 201 having the freshwater inflow port 210 and the second surface 12 of the solar cell panel 10 may be connected to each other to form the space S into which the freshwater LC1 flows.

In particular, the first frame 201 may be brought into contact with the second surface 12 along a perimeter area of the second surface 12, and may be formed to a predetermined thickness T to form the space S into which the freshwater LC1 flows.

Accordingly, when the freshwater LC1 flows into the freshwater inflow portion 250, the freshwater LC1 may be in contact with the second surface 12 of the solar cell panel.

That is, when the freshwater LC1 flows into the freshwater inflow portion 250 through the freshwater inflow port 210, the freshwater LC1 is in contact with the second surface 12.

Since the second surface 12 of the solar cell panel is raised in temperature by light received through the light receiving portion of the first surface 11 of the solar cell panel, it is possible to improve the performance of the solar cell panel 10 by cooling the solar cell panel 10 through heat exchange with the influent freshwater LC1 as described above.

Thus, the influent freshwater LC1 as described above is raised in temperature by the second surface 12 of the solar cell panel.

The freshwater with a higher temperature than the freshwater LC1 first introduced may flow to the first flow path 241.

Due to such contact, the freshwater may convect at the freshwater inflow portion 250 by a temperature difference between the freshwater and the second surface 12.

Meanwhile, the salinity gradient power generation unit 20 additionally includes a first inflow member 260 provided to distribute the freshwater LC1 flowing into the freshwater inflow portion 250 to the first flow path 241 and to uniformly distribute the freshwater LC1 to the plurality of ion exchange membranes 240 while without flowing to the second flow path 242.

That is, the first inflow member 260 may be provided to distribute the freshwater flowing into the freshwater inflow portion 250 to flow into a plurality of first flow paths while without flowing to the second flow path 242.

In more detail, the first inflow member 260 may be provided between the freshwater inflow portion 250 and the plurality of ion exchange membranes 240.

The first inflow member 260 may have a plurality of holes having a predetermined size through which freshwater passes to flow to the first flow path.

As an example, the first inflow member 260 may be a perforated panel, but the present invention is not limited thereto.

In particular, the first inflow member 260 includes a first perforated panel 261 having a plurality of first holes 261a and a second perforated panel 262 having a plurality of second holes 262a smaller than the first holes 261. That is, the first holes 261a have a larger size than the second holes 262a.

The first perforated panel 261 and the second perforated panel 262 may be sequentially arranged with respect to the freshwater inflow portion 250. By passing the freshwater LC1 through the first holes 261a with a larger size and then the second holes 262a with a smaller size as described above, the freshwater LC1 may efficiently, quickly, and uniformly flow to the first flow path 241. Here, as described above, the first inflow member 260 includes a first perforated panel and a second perforated panel. However, the present invention is not limited thereto, and it should be understood that only a first perforated panel may be included or a third perforated panel may be additionally included.

Meanwhile, due to a salinity gradient between the freshwater flowing through the first flow path 241 and the saltwater flowing through the second flow path 242, ionic substances contained in the saltwater selectively pass through the plurality of ion exchange membranes 240 to produce electricity.

In more detail, referring to FIG. 13, when the first freshwater flowing from the first freshwater inflow portion 250 and the first saltwater flowing through the first saltwater inflow port 213 flow through the first flow path 241 and the second flow path 242, ionic substances, i.e., a cationic substance and an anionic substance contained in the first saltwater having a higher salt concentration than the first freshwater selectively pass through the cation exchange membrane C and the anion exchange membrane A. Thus, an electric potential difference is generated, and an oxidation reaction and a reduction reaction occur at the anode 220 and the cathode 230 to generate a flow of electrons. As a result, electricity may be produced. As an example, the cationic substance may be a sodium ion (Na+), and the anionic substance may be a chlorine ion (Cl-), but the present invention is not limited thereto.

In particular, a shielding cation exchange membrane may be used as the ion exchange membranes disposed adjacent to the anode 220 and the cathode 230, for example, a cation exchange membrane and may be an ion exchange membrane with a higher monovalent ion selectivity. On the other hand, this is the same for an anion exchange membrane.

Referring to FIG. 12B, the plurality of ion exchange membranes 240 may additionally include a spacer 243 and a gasket 244 to form the first flow path 241 and the second flow path 242. The spacer 243 and the gasket 244 may be provided for each of a plurality of cation exchange membranes C and anion exchange membranes A.

As shown in FIG. 12A, by sequentially stacking and alternately arranging the cation exchange membrane C and the anion exchange membrane A each including the spacer 243 and the gasket 244 between the anode 220 and the cathode 230 formed at both distal ends, a plurality of first flow paths 241 and a plurality of second flow paths 242 may be formed.

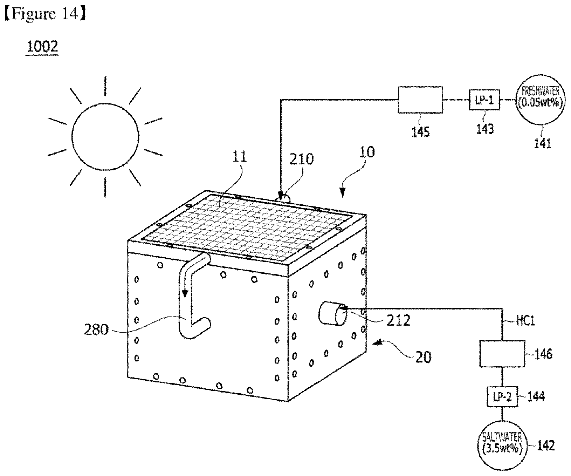

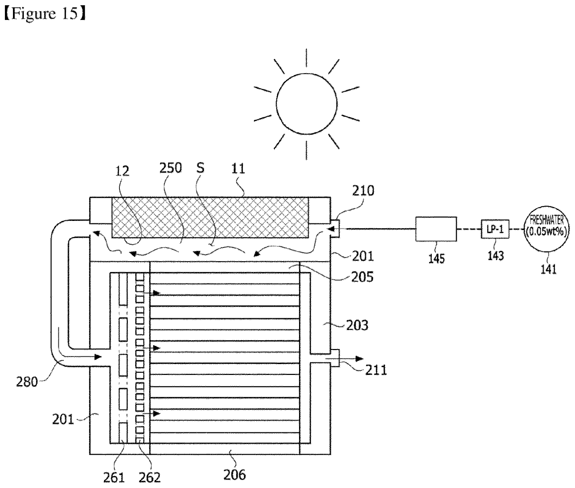

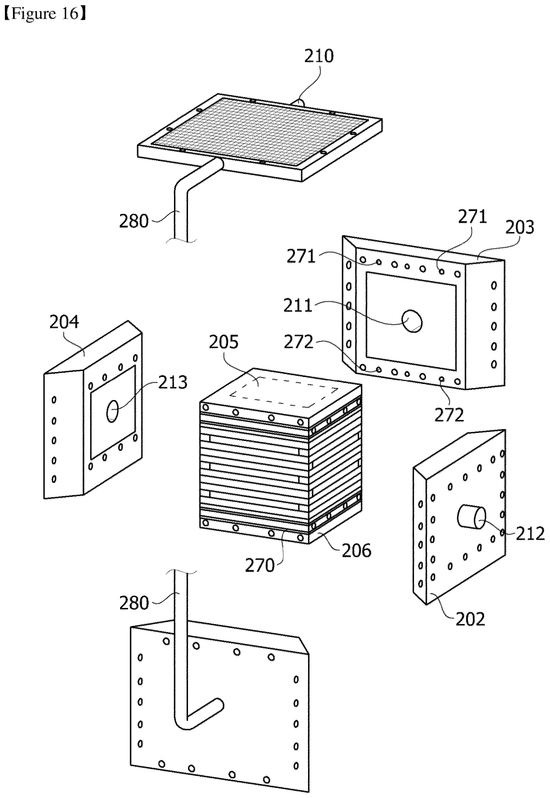

FIG. 14 is a perspective view of a second embodiment 1002 of the rear-cooling-integrated hybrid power generation apparatus, FIG. 15 is a side view of FIG. 14, and FIG. 16 is an exploded perspective view of FIG. 14.

Referring to FIGS. 14 to 16, the salinity gradient/solar energy hybrid power generation apparatus 1002 according to the second embodiment of the present invention may additionally include a first connection flow path 280 configured to fluidly connect the freshwater inflow portion 250 and the first inflow member 260 to deliver freshwater toward the first flow path.

In more detail, as described above, the freshwater flowing into the freshwater inflow portion 250 is brought into contact with the second surface of the solar cell panel 10 and raised in temperature. The freshwater raised in temperature may flow to the first flow path 241 through the first connection flow path 280.

As shown in FIG. 15, when the freshwater inflow portion 250 includes the first connection flow path 280, the freshwater inflow portion 250 may be connected to and in contact with the fifth frame 205.

Thus, the first frame 201 may be connected to and in contact with one side surfaces of the second to fourth frames 202, 203, and 204.

A process of producing electricity using the salinity gradient/solar energy hybrid power generation apparatus 1001 or 1002 having the above configuration (the first and second embodiments of the rear-cooling-integrated hybrid power generation apparatus) will be described below with reference to FIGS. 9 to 16.

First, as shown in FIGS. 9 and 16, when sunlight is incident on the first surface 11 having the light receiving portion of the solar cell panel 10, electricity is produced by the incident sunlight. In this case, when electricity is produced, the temperature of the first surface 11 is increased by the sunlight incident on the first surface 11, and the temperature of the second surface 12 is also increased due to conduction.

The freshwater flowing to the freshwater inflow portion 250 through the freshwater inflow port 210 is brought into contact with the second surface 12 raised in temperature as described above and thus is raised in temperature. That is, by the solar cell panel 10 being cooled by freshwater, efficiency is improved in producing electricity using sunlight.

As described above, the freshwater raised in temperature as described above flows to the first flow path 241 through the first inflow member 260. When the freshwater flows into the first flow path 241 as described above, saltwater flows to the second flow path 242 through the saltwater inflow port 212. The influent freshwater and saltwater as described above may flow through the first flow path 241 and the second flow path 242, and electricity may be produced due to a salinity gradient between the freshwater and the saltwater.

That is, when a cationic substance and an anionic substance contained in the saltwater having a higher salt concentration than the freshwater selectively pass through the cation exchange membrane C and the anion exchange membrane A, an electric potential difference may be generated to produce electricity. In this case, the moving speed of the ionic substances increases due to the high temperature of the freshwater LC1, and thus it is possible to increase electricity production efficiency.

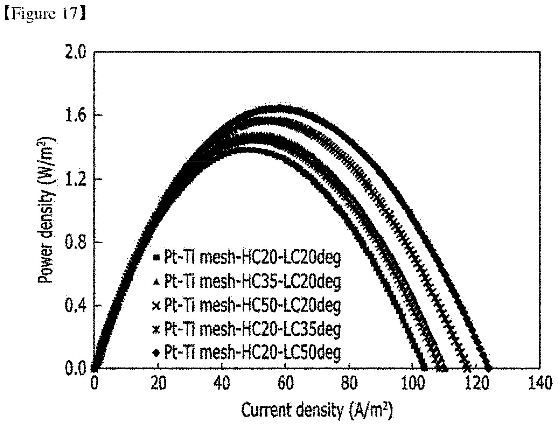

FIG. 17 is a graph showing power produced according to embodiments of the rear-cooling-integrated hybrid power generation apparatus.

Referring to FIG. 17, by introducing the freshwater raised in temperature to the first flow path 241 as described above, the moving speed of the ionic substances of the saltwater is increased to improve power production. The experimental conditions of FIG. 17 showing the result of performing the experiment according to the above embodiment are shown in Table 1 below.

**[Table 1]**

| | |
|---|---|
| High concentration (HC) | simulated seawater (NaCl 3.0 wt%) |
| Low concentration (LC) | Distilled water (NaCl 0.005 wt%) |
| Electrode rinse solution (ERS) | Ferricianide |
| Flow rates | - High concentration: 100 ml.min⁻¹ |
| | - Low concentration: 100 ml.min⁻¹ |
| | - Electrode rinse solution (ERS): 100 ml.min⁻¹ |
| Electrodes | Pt/Ti mesh |
| Spacer | 0.1 mm (Sefar) |
| Gasket | 0.1 mm (PTFE) |
| Effective area | D 5cm |
| Membranes | KEIR |

In the above experiment, NaCl salt simulation solution equivalent to 3.5 wt% of seawater was supplied as saltwater (high concentration solution) HC, and NaCl salt simulation solution equivalent to 0.005 wt% of freshwater was supplied as freshwater (low concentration solution) LC in the amount of 100 cc/min respectively. A KIER separation membranes produced by Korea Institute of Energy Research was used as the separator. As a result, the output power density is more improved by an increase in temperature at the freshwater side than by an increase in temperature at the saltwater side.

This shows that it is more advantageous, in terms of the improvement of the output power, to utilize freshwater LC rather than saltwater HC than to use both of the freshwater LC and the saltwater HC when a solution supplied to the salinity gradient power generation unit is selected in order to overcome a reduction in performance due to an increase in temperature of the solar photovoltaic power generation and/or solar thermal power generation unit 120 while the salinity gradient power generation unit 130 and the solar photovoltaic power generation and/or solar thermal power generation unit 120 perform hybrid power generation. Also, considering the stability of the solar photovoltaic power generation and/or solar thermal power generation unit 120 against corrosion caused by salt in addition to the improvement of the output power, it is appropriate to use the freshwater LC rather than the saltwater HC as cooling water of the solar photovoltaic power generation and/or solar thermal power generation unit 120. That is, considering both the performance improvement due to cooling and the stability of the apparatus, it is determined that it is advantageous to achieve the performance improvement due to cooling and the power production improvement due to an increase in temperature of a solution supplied to the salinity gradient power generation unit 130 by supplying the freshwater LC to the solar photovoltaic power generation and/or solar thermal power generation unit 120.

When power is produced as described above, power production efficiency may be increased by about 20% or more compared to a general RED apparatus.

As shown in FIG. 17, when freshwater LC1 having a temperature of 20°C flowed to the first flow path 241, the maximum power density was measured to be 1.4 W/m² per unit area of the ion exchange membrane. However, when freshwater LC1 was raised in temperature to 50°C and then introduced like the present invention, the maximum power density was measured to be 1.7 W/m² per unit area of the ion exchange membrane. That is, it may be ascertained that the power density is increased as described above.

After electricity is produced as described above, the freshwater and the saltwater may be discharged to the outflow ports 211 and 213.

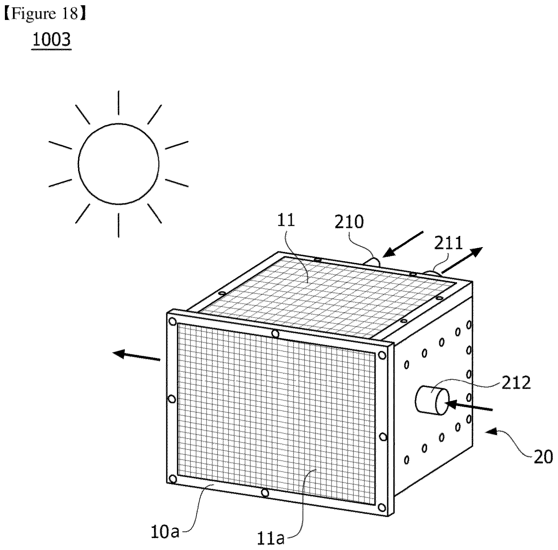

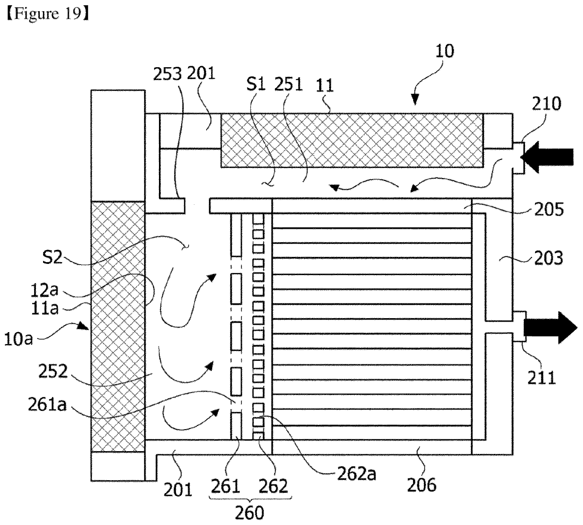

FIGS. 18 and 19 are diagrams showing a third embodiment 1003 of a rear-cooling-integrated hybrid power generation apparatus. FIG. 18 shows a perspective view of the third embodiment 1003, and FIG. 19 shows a cross-sectional view of FIG. 18.

Referring to FIGS. 18 and 19, the hybrid power generation apparatus according to the third embodiment 1003 is different from that of the first embodiment 1001 or the second embodiment 1002 in that one solar cell panel is additionally included. The other elements of the third embodiment 1003 are substantially the same as those of the first embodiment 1001 or the second embodiment 1002.

Referring to FIG. 18, the salinity gradient/solar energy hybrid power generation apparatus 1003 according to the third embodiment 1003 comprises a first solar cell panel 10 having a first surface 11 having a first light receiving portion and a second surface 12 opposite to the first surface.

In addition, the salinity gradient/solar energy hybrid power generation apparatus 1003 includes a second solar cell panel 10a having a first surface 11a having a second light receiving portion and a second surface 12a opposite to the first surface.

Also, the salinity gradient/solar energy hybrid power generation apparatus 1003 includes a salinity gradient power generation unit 20 for receiving freshwater and saltwater and producing electricity through salinity gradient power generation.

In more detail, the salinity gradient power generation unit 20 includes a housing 200 having a freshwater inflow port 210, a freshwater outflow port 211, a saltwater inflow port 212, and a saltwater outflow port 213.

Also, the salinity gradient power generation unit 20 includes an anode 220 and a cathode 230 placed in the housing 200 and spaced a predetermined distance from each other and a plurality of ion exchange membranes 240 arranged between the anode 220 and the cathode 230 to divide a first flow path 241 through which freshwater flows and a second flow path 242 through which saltwater flows. Here, the plurality of ion exchange membranes 240 includes a cation exchange membrane C and an anion exchange membrane A.

Also, the housing 200 may have a freshwater inflow portion 250 configured to fluidly connect the freshwater inflow port 210 and the first flow path 241. In particular, depending on the flowing direction of freshwater, the freshwater inflow portion 250 may be provided to achieve heat exchange between the second surface 12 of the first solar cell panel 10 and the freshwater and then heat exchange between the second surface 12a of the second solar cell panel 10a and the freshwater. Here, the freshwater inflow portion 250 includes a first inflow portion 251 connected to the freshwater inflow port 210 and a second inflow portion 252 connecting the first inflow unit 251 to the first flow path 241.

That is, when freshwater flows into the first inflow portion 251, the freshwater comes into contact with the second surface of the first solar cell panel, flows into the second inflow portion 252 according to the flowing direction of the freshwater, and then comes into contact with the second surface 120 of the second solar cell panel 100. Here, the flowing direction of the freshwater may be a direction in which freshwater may flow along the length direction of the first inflow portion and then flow into the second inflow portion 252.

The second inflow portion 252 may include an inflow port 253 into which the freshwater flows from the first inflow portion 251. Accordingly, the freshwater may flow into the first inflow portion and then flow into the second inflow portion 252 through the inflow port 253.

Also, the first and second solar cell panels additionally include heat dissipation members 13 provided in at least portions of the second surfaces 12 and 12a respectively.

In addition, the salinity gradient power generation unit 20 according to the third embodiment 1003 additionally includes a first inflow member 260 disposed in the second inflow portion 252 and configured to distribute freshwater to the first flow path and also uniformly distribute the freshwater to the plurality of ion exchange membranes 240 while without flowing into the second flow path 242. That is, the first inflow member 260 may be provided to distribute the freshwater flowing into the freshwater inflow portion 250 to flow into a plurality of first flow paths while without flowing to the second flow path 242.

Also, due to a salinity gradient between the freshwater flowing through the first flow path 241 and the saltwater flowing through the second flow path 242, ionic substances contained in the saltwater selectively migrate through the plurality of ion exchange membranes to produce electricity.

Meanwhile, at the first and second inflow portions 251 and 252, the freshwater may be raised in temperature by the second surfaces 12 and 12a of the first and second solar cell panels.

In more detail, the first inflow portion 251 may have at least one surface forming a space S1 into which the freshwater flows, wherein the surface is the second surface 12 of the first solar cell panel. Also, the second inflow portion 252 may have at least one surface forming a space S2 into which the freshwater flows, wherein the surface is the second surface 12a of the second solar cell panel. Accordingly, the freshwater flowing into the freshwater inflow port 210 may be raised in temperature by coming into contact with the second surface 12 of the first solar cell panel, which is the at least one surface of the first inflow portion 251.

The freshwater raised in temperature as described above may flow into the second inflow portion 252 through the inflow port 253 and come into contact with the second surface 12a of the second solar cell panel, which is the at least one surface of the second inflow portion 252. Thus, the freshwater may be raised in temperature once more.

The freshwater raised in temperature may flow into the first flow path as described above and thus salinity gradient power generation may be efficiently achieved. In this case, the first and second solar cell panels 10 and 10a cooled by the influent freshwater as described above may have electricity production efficiency improved by a cooling effect.

A process of producing electricity using the salinity gradient/solar energy hybrid power generation apparatus 1003 having the above configuration will be described below with reference to FIG. 19.

First, when sunlight is incident on the first surface 11 having the light receiving portion of the first solar cell panel 10, electricity is produced by the incident sunlight. In this case, when electricity is produced, the temperature of the first surface 11 is increased by the sunlight incident on the first surface 11, and the temperature of the second surface 12 is also increased due to conduction.

The freshwater flowing to the first inflow portion 251 through the freshwater inflow port 210 is brought into contact with the second surface 12 of the first solar cell panel raised in temperature as described above and thus is raised in temperature while the second surface 12 is lowered in temperature.

That is, by the first solar cell panel 10 being cooled by freshwater, efficiency is improved in producing electricity using sunlight.

As described above, the freshwater raised in temperature flows to the second inflow portion 252 through the inflow port 253. Also, when sunlight is incident on the first surface 11a having the light receiving portion of the second solar cell panel 10a, electricity is produced by the incident sunlight. When the electricity is produced, the temperature of the first surface 11a is increased by the sunlight incident on the first surface 11a, and the temperature of the second surface 12a is also increased due to conduction. In this case, the freshwater flowing to the second inflow portion 252 is brought into contact with the second surface 12a of the second solar cell panel raised in temperature as described above and thus is raised in temperature while the second surface 12a is lowered in temperature.

That is, by the second solar cell panel 10a being cooled by freshwater, efficiency is improved in producing electricity using sunlight.

The freshwater raised in temperature as described above flows to the first flow path 241 through the first inflow member 260. When the freshwater flows into the first flow path as described above, saltwater flows to the second flow path 242 through the saltwater inflow port 212.

The influent freshwater and saltwater as described above may flow through the first flow path 241 and the second flow path 242, and electricity may be produced due to a salinity gradient between the freshwater and the saltwater. That is, when a cationic substance and an anionic substance in the saltwater having a higher salt concentration than the freshwater selectively pass through the cation exchange membrane C and the anion exchange membrane A, an electric potential difference may be generated to produce electricity. In particular, by introducing the freshwater raised in temperature to the first flow path 241 as described above, the moving speed of the ionic substances of the saltwater is increased to improve power production efficiency.

After electricity is produced as described above, the freshwater and the saltwater may be discharged to the outflow ports 211 and 213.

More details of the hybrid power generation apparatus are disclosed in detail in KR 10-2018-0093208 filed by the same applicant as the present applicant, the contents of which are incorporated therein.

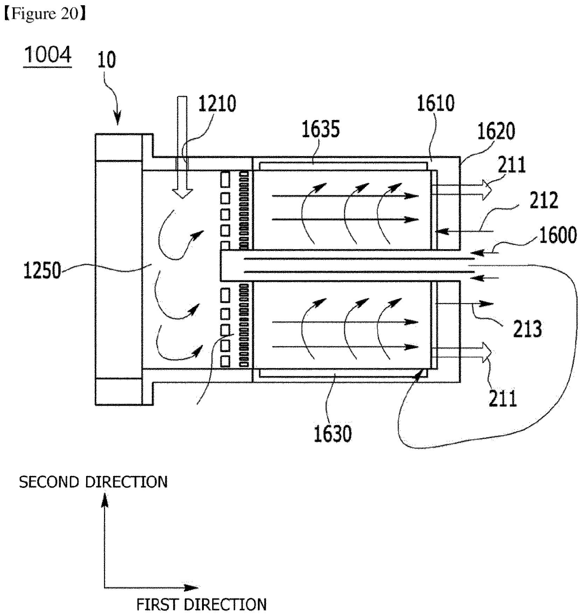

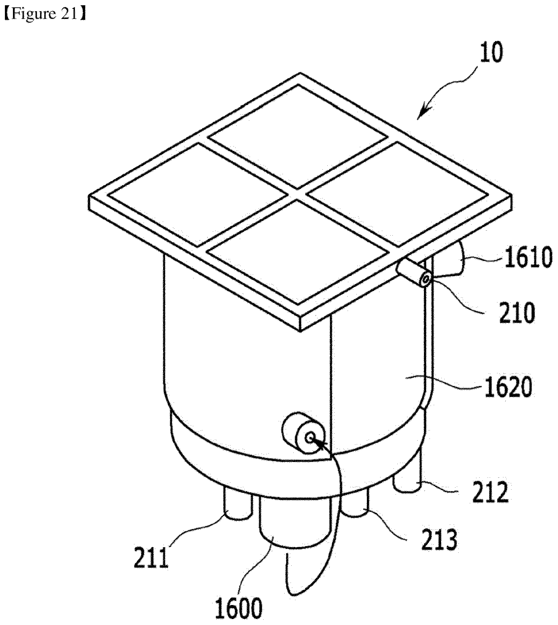

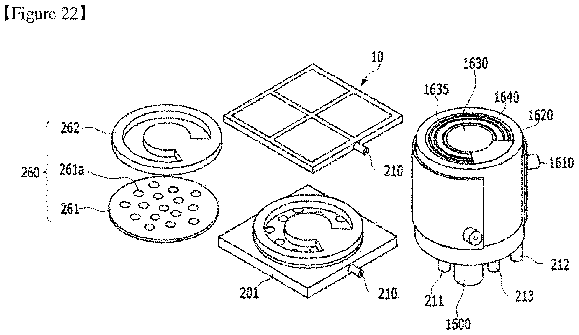

FIGS. 20 to 22 are diagrams showing a fourth embodiment 1004 of a rear-cooling-integrated hybrid power generation apparatus. FIG. 20 shows a cross-sectional view of the fourth embodiment 1004, FIG. 21 shows a perspective view of FIG. 20, and FIG. 22 shows an exploded perspective view of FIG. 20.

Referring to FIGS. 20 to 22, a salinity gradient power generation unit according to the fourth embodiment 1004 is different from that of the first embodiment 1001 or the second embodiment 1002 in that it is formed in a cylinder shape. The other elements of the fourth embodiment 1004 are substantially the same as those of the first embodiment 1001 or the second embodiment 1002.

The cylindrical salinity gradient power generation unit comprises a main body 1620 formed in a shape that is long in a first direction perpendicular to the first surface of the solar cell panel 10 and that is round in a second direction, that is, a circumferential direction. An electrode unit including a first electrode 1630 formed to be long in the first direction and a second electrode 1635 spaced a predetermine distance from the first electrode 1630 and formed to be round along the second direction is disposed in the main body 1620. An ion exchange membrane portion 1640 is placed between the first electrode 1630 and the second electrode 1635 and composed of a cation exchange membrane and an anion exchange membrane stacked in a round shape around the first electrode 1630 along the second direction.

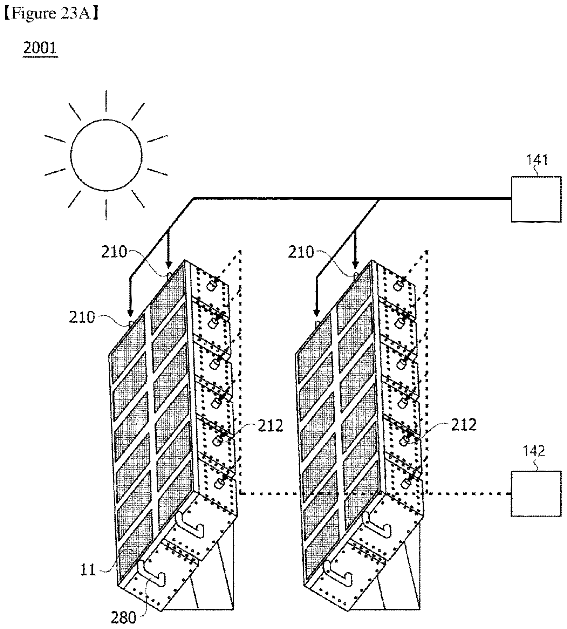

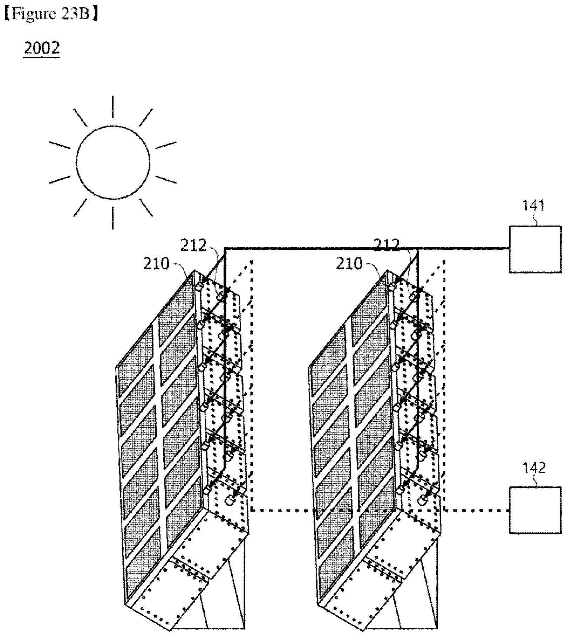

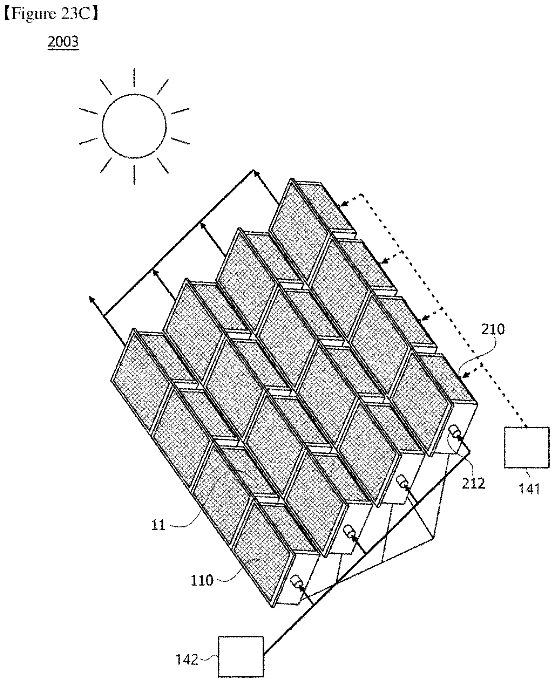

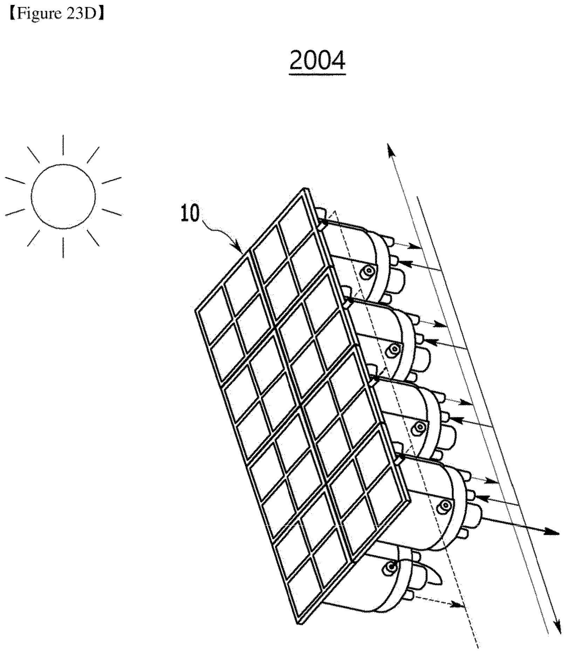

FIGS. 23A to 23D illustrate hybrid power generation systems 2001, 2002, 2003, and 2004 provided by connecting a plurality of rear-cooling-integrated hybrid power generation apparatuses according to the first to fourth embodiments 1001 to 1004.

Therefore, the detailed description of the salinity gradient/solar energy hybrid power generation apparatuses 1001, 1002, 1003, and 1004 may be applied to the hybrid power generation systems 2001, 2002, 2003, and 2004 to be described below.

By each of the hybrid power generation systems 2001, 2002, 2003, and 2004 including a plurality of hybrid power generation apparatuses 1001, 1002, 1003, and 1004, it is possible to efficiently produce power and thus improve power production.

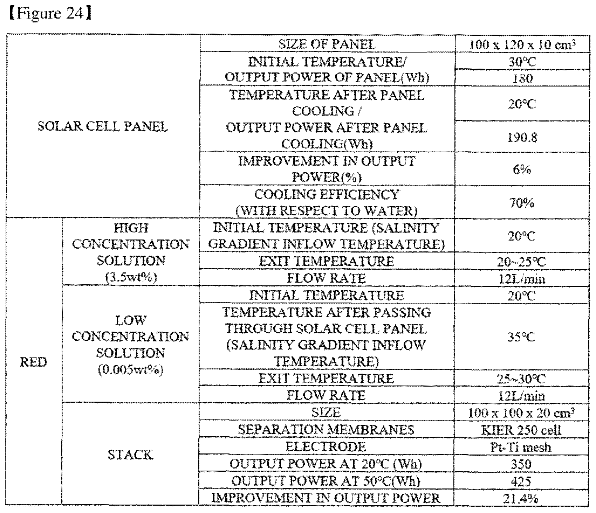

FIG. 24 is a diagram obtained by calculating an output power improvement rate of a hybrid power generation system according to a change in temperature on the basis of the embodiment of FIG. 17 in the salinity gradient/solar energy hybrid power generation apparatus shown in FIG. 14.

When the temperature at the freshwater side increased from 20°C to 50°C as shown in FIG. 17, the output power density of the salinity gradient power generation unit was increased from 1.4 W/m² to 1.7 W/m².

When this was applied to a salinity gradient power generation stack having a size of 100 X 100 X 20 cm³ (W X L X h) as shown in FIG. 24 (KIER separation membranes: about 250 cell pairs), the output power was calculated as increasing from about 350 Wh to 425 Wh by about 21.4%.

When the salinity gradient/solar energy hybrid power generation apparatus is configured as shown in FIG. 14 on the basis of the calculation, a solar cell panel having a size of 100 X 120 X 10 cm³ (W X L X h) may be configured. Assuming that cooling efficiency was about 70%, the output power increased from 180 Wh to 191 Wh by about 6% when the temperature of the solar cell panel was cooled from 30°C to 20°C.

When the supply source of the salinity gradient power generation was utilized as cooling water of the solar cell panel through this embodiment, both of the performance of the salinity gradient power generation and the performance of the solar cell power generation were improved.

Therefore, it can be seen that it is possible to optimize performance improvement rate control and the amount of power generation of a hybrid power generation apparatus through engineering design of a hybrid power generation structure using a solar cell panel by controlling the size, the supply amount, etc. of a salinity gradient power generation stack.

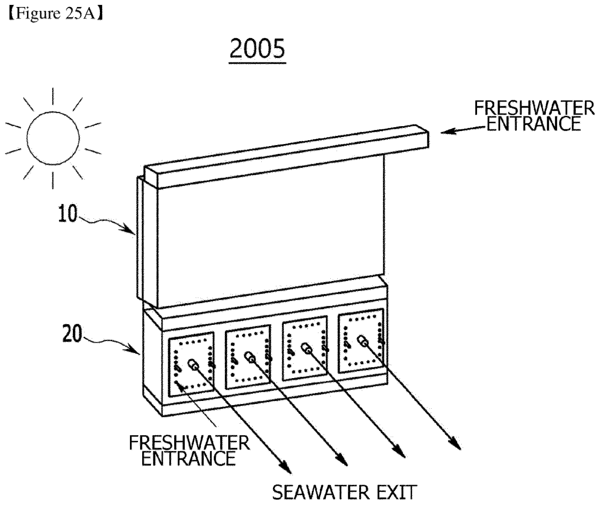

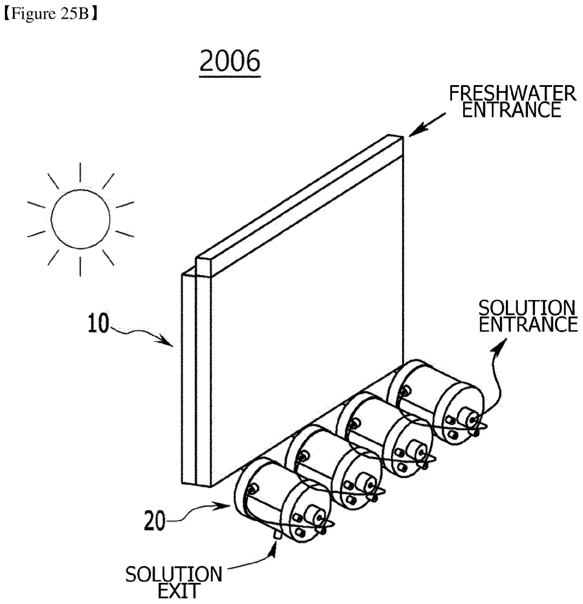

Meanwhile, FIGS. 25A and 25B show hybrid power generation systems 2005 and 2006 provided by connecting a plurality of salinity gradient power generation units 20 to the solar cell panel 10. By including a plurality of the above-described hybrid power generation apparatuses, it is possible to more efficiently produce power and thus improve power production.

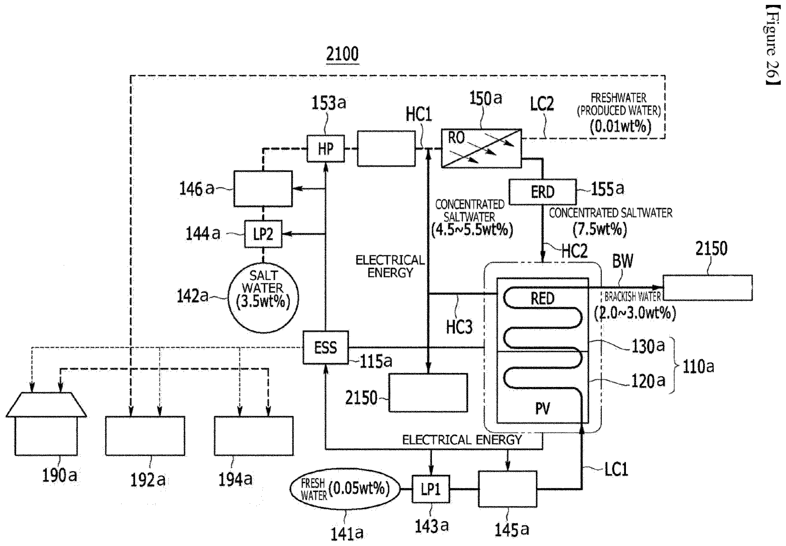

FIG. 26 shows a schematic diagram of a marine microalgae culture system 2100 using the hybrid power generation apparatus 110a according to the second embodiment. In FIG. 26, the concentration of concentrated seawater HC2 passing through the desalination unit 150a is from about 7.5 wt% to 8.0 wt%. When the concentrated water flows to the outside through the salinity gradient power generation unit 130a, the concentration is diluted to about 4.5 to 5.5 wt% (HC3), and on the contrary, the concentration of freshwater LC1 supplied to the salinity gradient power generation unit 130a is increased to about 2.0 to 3.0 wt%.

The effluent water LC1 and the effluent water HC3 flowing out after being supplied to the salinity gradient power generation unit 130a have salt concentrations that are comparable to or slightly higher than that of seawater. The effluent water LC1 and the effluent water HC3 contain enough nutrients to culture microalgae that live in the ocean. Therefore, as shown in FIG. 26, the effluent water may be supplied to a marine microalgae culture device 2150 as culture solution. When the hybrid power generation apparatus 110a is used, a marine microalgae growth pattern may be controlled because the temperature of water supplied to the marine microalgae culture device 2150 can be adjusted.

The seawater desalination system that has been described with reference to FIGS. 1 to 26 includes a salinity gradient/solar energy hybrid power generation apparatus. By using cooling water as a source of the salinity gradient power generation unit to suppress an increase in temperature of the power generation unit caused during a process of generating power using solar energy, the hybrid power generation apparatus may minimize a decrease in output power caused by an increase in temperature of the solar energy power generation unit and induce the improvement in output power of salinity gradient power generation caused by an increase in temperature of a supply source, and it is possible to obtain a synergy effect of improving the performance of hybrid power generation. Through the hybrid power generation, it is possible to minimize the area of a site for power generation and maximize the amount of power generation, thereby reducing a significant amount of energy used in a reverse osmosis process. The hybrid power generation apparatus may be installed both at the front stage and at the rear stage of a reverse osmosis seawater desalination apparatus and thus may be utilized as an environmentally friendly power generation source for concentrated wastewater. Also, the salinity gradient/solar energy hybrid power generation apparatus may use effluent water discharged after power generation as a source for culturing marine microalgae and thus may be effectively utilized in producing useful resources.

Meanwhile, a power generation system 3000 using the salinity gradient power generation apparatus according to an embodiment of the present invention includes a reverse electrodialysis salinity gradient power generator configured to produce electricity using freshwater and saltwater by including a reverse electrodialysis stack having a cation exchange membrane and an anion exchange membrane; and a first ion filter connected to an input stage of the reverse electrodialysis salinity gradient power generator and configured to filter out organic matter of the saltwater or freshwater flowing into the reverse electrodialysis salinity gradient power generator.

Also, the power generation system 3000 further includes a pre-treatment unit configured to remove floating substances contained in saltwater or freshwater supplied to the reverse electrodialysis salinity gradient power generator.

Also, the pre-treatment unit may be a cartridge filter.

Also, the cartridge filter may have a pore size of 5 µm or more.

Also, the first ion filter may be an anion exchange filter.

Also, the first ion filter may have a particle size of 100 µm to 1.2 mm.

Also, the power generation system 3000 further includes a second ion filter connected to the input stage of the reverse electrodialysis salinity gradient power generator, connected in parallel to the first ion filter, and configured to filter out organic matter of saltwater or freshwater flowing into the reverse electrodialysis salinity gradient power generator.

Also, the power generation system 3000 further includes a first selection valve configured to selectively provide the saltwater or freshwater to the first ion filter or the second ion filter.

Also, the first selection valve may be driven by a total organic carbon gradient of saltwater or freshwater flowing into and out of the first and second ion filters.

Also, the first selection valve may be driven by a pressure gradient of saltwater or freshwater flowing into and out of the first and second ion filters or by a pressure gradient of saltwater or freshwater flowing into and out of the reverse electrodialysis salinity gradient power generator.

Also, the power generation system 3000 further includes a post-treatment solution provision unit configured to provide a post-treatment solution for cleaning the first and second ion filters; and a second selection valve configured to selectively provide the post-treatment solution provided from the post-treatment solution provision unit to the first ion filter or the second ion filter.

In addition, a power generation system 3001 using the salinity gradient power generation apparatus according to an embodiment of the present invention includes a solar photovoltaic power generation apparatus configured to perform solar photovoltaic power generation by including a solar photovoltaic module, installed for influent saltwater or freshwater to pass through a surface of the solar photovoltaic module or a rear surface of the solar photovoltaic module, and configured to use the saltwater or freshwater so as to cool the temperature of the solar photovoltaic module; a reverse electrodialysis salinity gradient power generator configured to produce electricity using freshwater and saltwater by including a reverse electrodialysis stack having a cation exchange membrane and an anion exchange membrane; and a first ion filter connected to an input stage of the reverse electrodialysis salinity gradient power generator and configured to filter out organic matter of the saltwater or freshwater flowing into the reverse electrodialysis salinity gradient power generator.

Also, the solar photovoltaic module may be a floating solar photovoltaic panel that floats on the surface of the saltwater or the freshwater.

Also, wastewater discharged after the reverse electrodialysis salinity gradient power generator performs salinity gradient power generation may be introduced into a small hydro power generator or a tidal current power generator.

Also, the first ion filter and the reverse electrodialysis stack may be configured in an integrated manner.

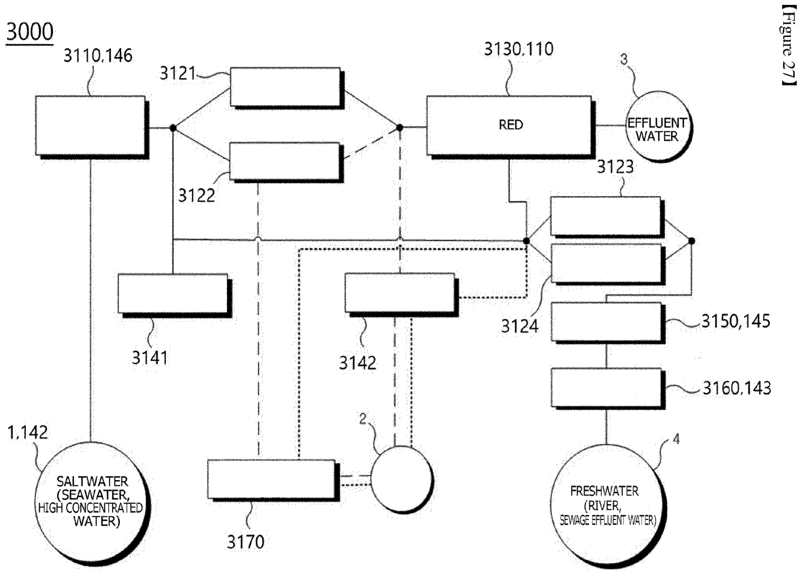

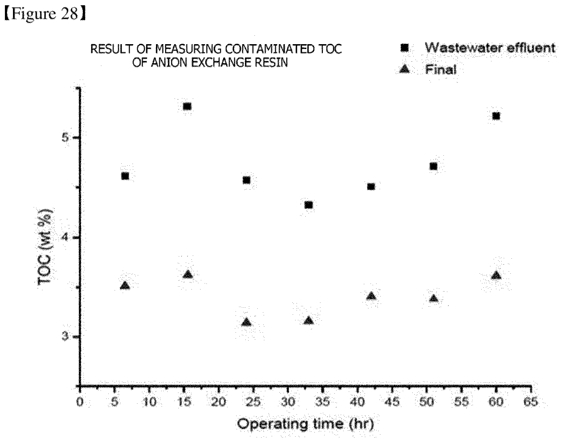

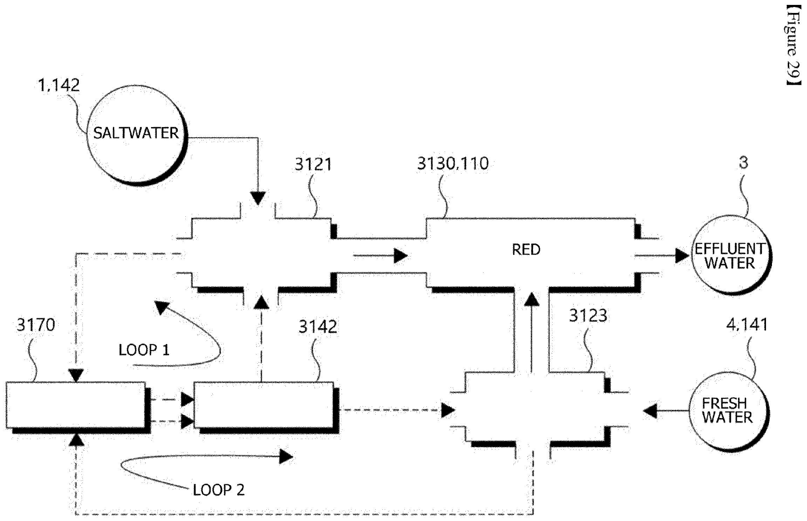

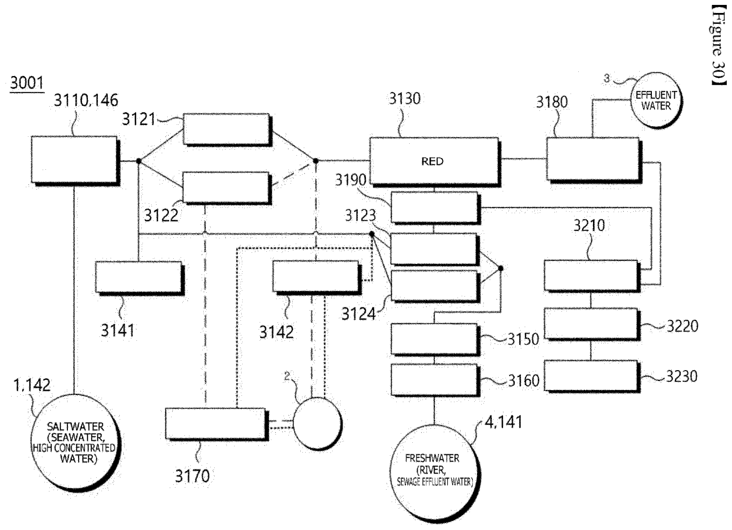

FIG. 27 is a block diagram showing a schematic structure of the power generation system 3000 using salinity gradient power generation according to an embodiment of the present invention, FIG. 28 is a graph showing a contaminated total organic carbon (TOC) before and after freshwater passes through third and fourth ion filters in the power generation system using the salinity gradient power generation according to an embodiment of the present invention, FIG. 29 is a block diagram showing operation of the ion filter and the reverse electrodialysis salinity gradient power generator in the power generation system using the salinity gradient power generation according to an embodiment of the present invention, and FIG. 30 is a block diagram showing a schematic structure of the power generation system using salinity gradient power generation according to an embodiment of the present invention.

Referring to FIGS. 27 to 30, the power generation system 3000 using salinity gradient power generation may include a saltwater pre-treatment unit 3110, first to third ion filters 3121, 3122, and 3123, a reverse electrodialysis (RED) salinity gradient power generator 3130, first and second selection valves 3141 and 3142, a freshwater pre-treatment unit 3150, a low-pressure pump unit 3160, and a post-treatment solution provision unit 3170.

The saltwater pre-treatment unit 3110 receives and pre-treats saltwater 1, which is seawater or high-concentrated water, and delivers the pre-treated saltwater to the RED salinity gradient power generator 3130. The saltwater pre-treatment unit 3110 may be a cartridge filter having a pore size of 0.5 µm to 200 µm. In a preferred embodiment, the pore size is 5 µm so that foreign substances having sizes of 5 µm or greater are filtered out of floating substances of saltwater flowing into the RED salinity gradient power generator 3130.

The first and second ion filters 3121 and 3122 are cation exchange filters or anion exchange filters for exchanging cations or anions of saltwater that is pre-treated by the saltwater pre-treatment unit 3110 and used for salinity gradient power generation in the RED salinity gradient power generator 3130. The first and second ion filters 3121 and 3122 are connected in parallel to an input stage of the RED salinity gradient power generator 3130 and have a particle size of 100 µm to 1.2 mm.

In an example, the ion filters may be provided as the at least two ion filters, such as a pair of the first ion filter 3121 and the second ion filter 3122, as shown in FIG. 27. The driving of the first and second ion filters 3121 and 3122 is controlled by the first selection valve 3141 to introduce, or not to introduce, saltwater to the RED salinity gradient power generator 3130.

When the first and second ion filters 3121 and 3122 are anion filters, the first and second ion filters 3121 and 3122 remove organic matter contained in saltwater flowing into the RED salinity gradient power generator 3130. Thus, the anion exchange membranes of the RED salinity gradient power generator 3130 can overcome a disadvantage of a reduction in power generation performance of salinity gradient power generation due to contamination caused by organic matter. Also, there is no need to stop a power generator so as to replace the anion exchange membranes of the RED salinity gradient power generator 3130, and thus the power generation performance of the salinity gradient power generator may be improved.

When the first and second ion filters 3121 and 3122 are cation filters, the first and second ion filters 3121 and 3122 remove polyvalent ions (Mg²⁺, Ca²⁺) of saltwater flowing into the RED salinity gradient power generator 3130. Thus, the cation exchange membranes of the RED salinity gradient power generator 3130 can overcome a disadvantage of a reduction in performance due to polyvalent ions.

Thus, by the first and second ion filters 3121 and 3122 performing the operations of removing organic matter and polyvalent ions, it is possible to lengthen the post-treatment cycle of the stack of the RED salinity gradient power generator 3130, thereby reducing the maintenance cost of the RED salinity gradient power generator 3130.

Also, since the power generation is possible through continuous maintenance and cleaning by means of the first and second ion filters 3121 and 3122 without stopping the RED salinity gradient power generator 3130 for the purpose of stack post-treatment, it is possible to improve performance of salinity gradient power generation.

The first and second ion filters 3121 and 3122 may be formed in an integrated structure with the salinity gradient power generation stack of the RED salinity gradient power generator 3130. The third ion filter 3123 and a fourth ion filter 3124 are configured as anion filters to treat organic matter of freshwater 4 flowing into the RED salinity gradient power generator 3130 and include a plurality of ion filters in pairs as the first and second ion filters 3121 and 3122 are configured in pairs. In this case, the third and fourth ion filters 3123 and 3124 treat the organic matter of the freshwater 4. In an embodiment where the freshwater 4 is sewage effluent water, the contaminated TOC of freshwater after being treated with the third and fourth ion filters 3123 and 3124 is decreased by about 30% to 50% compared to the contaminated TOC of freshwater before flowing into the third and fourth ion filters 3123 and 312, as shown in FIG. 28. As described above, organic matter that may affect the stack of the salinity gradient power generator 130 may be treated by the third and fourth ion filters 3123 and 3124.

Also, the first selection valve 3141 is a selection valve configured to provide saltwater to the RED salinity gradient power generator 3130 through any one of the first and second ion filters 3121 and 3122 and configured to provide freshwater to the RED salinity gradient power generator 3103 through any one of the third and fourth ion filters 3123 and 3124 provided in pairs when the third ion filter 3123 is provided in pairs.

The first selection valve 3141 is driven to select any one of the first and second ion filters 3121 and 3122 according to a difference between the TOC of saltwater flowing into the first and second ion filters 3121 and 3122 and the TOC of saltwater flowing out of the first and second ion filters 3121 and 3122.

In an example, the first selection valve 3141 includes a treatment apparatus having a set reference value and is driven to monitor a difference of the TOC of saltwater flowing out of the first and second ion filters 3121 and 3122, compare the monitored difference in saltwater TOC to a reference value, and generate a selection signal for selecting any one of the first and second ion filters 3121 and 3122.

Also, the first selection valve 141 is driven to compare a difference between the TOC of freshwater flowing into the third and fourth ion filters 3123 and 3124 and the TOC of freshwater flowing out of the third and fourth ion filters 3123 and 3124 to a reference value, generate a selection signal for selecting any one of the third and fourth ion filters 3123 and 3124, and perform the selection.

In an example, the first selection valve 3141 is driven to select the first to fourth ion filters 3121, 3122, 3123, and 324 monitored to have a TOC difference of saltwater or freshwater flowing into and out of the first to fourth ion filters 3121, 3122, 3123, and 3124 being greater than or equal to 30%. Meanwhile, the first selection valve 3141 performs control to perform a post-treatment operation while blocking saltwater or freshwater from passing through an ion filter having a TOC difference of less than 30%.

As another example, the first selection valve 3141 is driven to select any one of the first and second ion filters 3121 and 3122 according to a difference in pressure between saltwater flowing into the first and second ion filters 3121 and 3122 and saltwater flowing out of the first and second ion filters 3121 and 3122 and any one of the third and fourth ion filters 3123 and 3124 according to a difference in pressure between saltwater flowing into the third and fourth ion filters 3123 and 3124 and saltwater flowing out of the third and fourth ion filters 3123 and 3124.

In an example, the first selection valve 3141 is driven to select a corresponding ion filter determined to have a difference in pressure between saltwater and freshwater flowing into the first to fourth ion filters 3121, 3122, 3123, and 3124 and saltwater and freshwater flowing out of the first to fourth ion filters 3121, 3122, 3123, and 3124 being less than or equal to 0.5 kgf/cm². Meanwhile, the first selection valve 3141 performs control to perform a post-treatment operation while blocking saltwater or freshwater from passing through an ion filter having a difference in pressure of greater than 0.5 kgf/cm².

Thus, by the first selection valve 3141 introducing saltwater to the RED salinity gradient power generator 3130 through any one of the first and second ion filters 3121 and 3122 or introducing freshwater to the RED salinity gradient power generator 3130 through any one of the third and fourth ion filters 3123 and 3124, it is possible to easily control the operation of an ion filter to be replaced, that is, to be post-treated. As another example, the first to fourth ion filters 3121, 3122, 3123, and 3124 or the first selection valve 3141 may monitor state information regarding the anion exchange filters of the first to fourth ion filters 3121, 3122, 3123, and 3124, generate notification information using the monitoring result, and deliver the notification information to an external manger terminal or management server. In this case, a communication component may be further included, and the present invention is not limited thereto.

The second selection valve 3142 is a valve for supplying a post-treatment solution 2 stored in the post-treatment solution provision unit 3170 to the first to fourth ion filters 3121, 3122, 3123, and 3124. As shown in FIG. 29, the second selection valve 3142 may be driven with a first loop in which the post-treatment solution 2 stored in the post-treatment solution provision unit 3170 passes through the first ion filter 3121 and returns to the post-treatment solution provision unit 3170 by means of the second selection valve 3142 and a second loop in which the post-treatment solution 2 stored in the post-treatment solution provision unit 3170 passes through the third ion filter 3123 and returns to the post-treatment solution provision unit 3170 by means of the second selection valve 3142.

Like the first selection valve 3141 being driven to select to provide saltwater or freshwater to the first to fourth ion filters 3121, 3122, 3123, and 3124, the second selection valve 3142 is driven to provide the post-treatment solution 2 to the first to fourth ion filters 3121, 3122, 3123, and 3124 according to the difference in TOC between saltwater or freshwater flowing into the first to fourth ion filters 3121, 3122, 3123, and 3124 and saltwater or freshwater flowing out of the first to fourth ion filters 3121, 3122, 3123, and 3124 or the difference in pressure between saltwater or freshwater flowing into the first to fourth ion filters 3121, 3122, 3123, and 3124 or the RED salinity gradient power generator 3130 and saltwater or freshwater flowing out of the first to fourth ion filters 3121, 3122, 3123, and 3124 or the RED salinity gradient power generator 3130.

By the second selection valve 3142 being driven to provide the post-treatment solution 2 stored in the post-treatment solution provision unit 3170 to the first to fourth ion filters 3121, 3122, 3123, and 3124 through the first and second loops, it is possible to maintain the state of the first to fourth ion filters 3121, 3122, 3123, and 3124 having performance degraded due to the filtering of floating substances and foreign substances and the exchange of anions.

In an example, the post-treatment solution provided to the first to fourth ion filters 3121, 3122, 3123, and 3124 may be a solution obtained by dissolving NaCl, NaOH, and Na₂CO₃ in water. In this case, it is preferred that NaCl be 1.0 M (mole) or less and NaOH be 0.01 M or less.

Also, in this case, when the post-treatment solution is supplied to the first to fourth ion filters 3121, 3122, 3123, and 3124 to be post-treated, air and the post-treatment solution may be simultaneously supplied. By simultaneously supplying air and the post-treatment solution to the first to fourth ion filters 3121, 3122, 3123, and 3124, the air may cause a bubble effect while the post-treatment solution chemically treats organic matter adsorbed to the ion filters. Thus, the chemically treated organic matter may be physically treated, and thus it is possible to maximize a cleaning effect.

As an example, the amount of air flowing into the first to fourth ion filters 3121, 3122, 3123, and 3124 may be supplied at 1 to 50 vol% of the post-treatment solution flowing into the first to fourth ion filters 3121, 3122, 3123, and 3124. The type and concentration of the post-treatment solution supplied to the first to fourth ion filters 3121, 3122, 3123, and 3124 and the amount of supplied air may be set variously depending on the operating conditions of the salinity gradient power generation, and the present invention is not limited thereto.

The structure of the power generation system 3000 using the salinity gradient power generation according to an embodiment of the present invention will be described with reference back to FIG. 27. The RED salinity gradient power generator 3130 receives freshwater 4, which is river and sewage effluent water, in addition to saltwater 1 and includes a freshwater pre-treatment unit 3150 configured to pre-treat the freshwater and a low-pressure pump unit 3160 configured to facilitate the inflow of the freshwater 4 flowing into the RED salinity gradient power generator 3130.

In this case, although not shown, the low-pressure pump unit 3160 capable of moving the freshwater 4 to the freshwater pre-treatment unit 3150 may be formed at an inflow stage of the saltwater pre-treatment unit 3110 configured to receive the saltwater 1. The present invention is not limited thereto.

The RED salinity gradient power generator 3130 performs RED salinity gradient power generation using the saltwater 1 pre-treated by the saltwater pre-treatment unit 3110 and the freshwater 4 pre-treated by the freshwater pre-treatment unit 3150 to produce electricity and discharges the saltwater 1 or freshwater 4 used to perform the RED salinity gradient power generation as effluent water 3.

The power generation system 3001 using salinity gradient power generation according to another embodiment of the present invention may further include a small hydro power generator/tidal current power generator 3180, a solar photovoltaic power generation apparatus 3190, an energy storage system 3210, a power conversion unit 3220, and an electricity charging system 3230. The small hydro power generator/tidal current power generator 3180 performs small hydro power generation or tide current power generation using saltwater or freshwater that has been used by the RED salinity gradient power generator 130 to perform salinity gradient power generation and then discharges the saltwater or freshwater as the effluent water 3.

By the solar photovoltaic power generation apparatus 3190 being located on a path along which the freshwater 4 pre-treated by the freshwater pre-treatment unit 3150 flows into the RED salinity gradient power generator 3130, the pre-treated freshwater cools the solar photovoltaic module constituting the solar photovoltaic power generation apparatus 3190 raised in temperature by daylight, and thus it is possible to improve power generation efficiency of the solar photovoltaic power generation apparatus 3190. Also, since the freshwater 4 flowing to the outside through the solar photovoltaic power generation apparatus 3190 is supplied to the salinity gradient power generator 3130 while raised in temperature, it is possible to obtain an effect of improving the salinity gradient power generation output of the salinity gradient power generator 4, as shown in Table 2 below.

**[Table 2]**

| Solar Photovoltaic Panel (Solar photovoltaic Power Generation and/or Solar Thermal Power Generation Unit) | Freshwater Temperature (RED Entrance, °C) | Seawater Temperature (RED Entrance, °C) | Current Density (A/m²) | Power Density (W/m²) |
|---|---|---|---|---|
| Absence | 20 | 20 | 48 | 1.4 |
| Presence | 30 | | 51 | 1.45 |
| | 40 | | 54 | 1.5 |
| | 50 | | 59 | 1.7 |

In another example, although not shown, the pre-treated saltwater as well as the pre-treated freshwater is structurally designed to pass through a flow path installed on the surface of the solar photovoltaic module of the solar photovoltaic power generation apparatus 3190 or on the rear surface of the solar photovoltaic power generation apparatus 3190 and thus may be configured to pass as cooling water. In this case, it is possible to improve the salinity gradient power generation output of the salinity gradient power generator 4 as shown in Table 3 below.

**[Table 3]**

| Solar Photovoltaic Panel (Solar | Seawater | Freshwater | Current | Power |
|---|---|---|---|---|
| photovoltaic Power Generation and/or Solar Thermal Power Generation Unit) | Temperature (RED Entrance, °C) | Temperature (RED Entrance, °C) | Density (A/m²) | Density (W/m²) |
| Absence | 20 | 20 | 48 | 1.4 |
| Presence | 30 | | 52 | 1.5 |
| | 40 | | 55 | 1.53 |
| | 50 | | 58 | 1.59 |

When the freshwater 4 having passed through the third and fourth ion filters 3123 and 3124 flows into the RED salinity gradient power generator 3130 via the solar photovoltaic power generation apparatus 3190, the third and fourth ion filters 3123 and 3124, the solar photovoltaic power generation apparatus 3190, and the RED salinity gradient power generator 3130 may be configured in an integrated manner. The solar photovoltaic power generation apparatus 3190 may be formed as a floating solar photovoltaic panel that is formed to float on the surface of saltwater or freshwater.

The energy storage system 3210 receives and stores electricity produced through small hydro power generation or tidal current power generation by the small hydro power generator/tidal current power generator 3180 or stores electricity produced using the solar photovoltaic module in the solar photovoltaic power generation apparatus 3190 or electricity produced through salinity gradient power generation in the RED salinity gradient power generator 3130.

The power conversion unit 3220 converts the electricity stored in the energy storage system 3210 and produced in the power generation system into power, and the electricity charging system 3230 implements power obtained through the conversion of the power conversion unit 220 with an electricity charging infrastructure such as an electricity charging station.

As indicated by reference numerals written in FIGS. 27 to 30, the elements in the power generation systems 3000 and 3001 using salinity gradient power generation, which will be sequentially disclosed, may be the same as those of the desalination system 100 according to the first embodiment.

The saltwater 1, the saltwater pre-treatment unit 3110, the freshwater 4, the low-pressure pump unit 3160, and the freshwater pre-treatment unit 3150 in the power generation systems 3000 and 3001 using salinity gradient power generation may be the first saltwater supply unit (second supply unit) 142, the second pre-treatment unit (pre-treatment 2) 146, the first freshwater supply unit (first supply unit) 141, the first pressure adjustment unit 143, and a first pre-treatment unit (pre-treatment 2) 143 of the desalination system 100 according to the first embodiment, respectively.

In addition, the RED salinity gradient power generator 3130 of the power generation system 3000 using salinity gradient power generation and the solar photovoltaic power generation apparatus 3190 and the RED salinity gradient power generator 3130 of the power generation system 3001 using salinity gradient power generation, which are configured in an integrated manner, may be the hybrid power generation apparatus 110 including the solar photovoltaic power generation and/or solar thermal power generation unit 120 and the salinity gradient power generation unit 130 described above, and more particularly, a rear-cooling-integrated hybrid power generation apparatus.

Accordingly, the detailed description of the power generation systems 3000 and 3001 using salinity gradient power generation may be applied to the hybrid power generation apparatus 110 including the solar photovoltaic power generation and/or solar thermal power generation unit 120 and the salinity gradient power generation unit 130 and the desalination systems 100, 100a, 100b, 100c, and 100d using the same. The detailed description of the hybrid power generation apparatus 110 including the solar photovoltaic power generation and/or solar thermal power generation unit 120 and the salinity gradient power generation unit 130 and the desalination systems 100, 100a, 100b, 100c, and 100d using the same may be applied to the power generation systems 3000 and 3001 using salinity gradient power generation.

## Claims

1. A salinity gradient and solar energy hybrid power generation apparatus comprising:
a solar photovoltaic power generation and/or solar thermal power generation unit (120), supplied with first freshwater as cooling water, which has a solar cell panel (10) having a first surface (11) having a light receiving portion and a second surface (12) opposite to the first surface, and
a salinity gradient power generation unit (130) supplied with first saltwater and the first freshwater raised in temperature due to heat exchange with the solar cell panel (10) to generate electricity by a salinity gradient between the first freshwater and the first saltwater, the salinity gradient power generation unit (130) comprises:
a housing (200) having a first freshwater inflow port (210), a first freshwater outflow port (211), a first saltwater inflow port (212), and a first saltwater outflow port (213);
an anode (220) and a cathode (230) disposed in the housing (200) and spaced a predetermined distance from each other; and
a plurality of ion exchange membranes (240) arranged between the anode (220) and the cathode (230) to compart a first flow path (241) through which the first freshwater flows and a second flow path (242) through which the first saltwater flows,
the housing (200) has a first freshwater inflow portion (250) configured to fluidly connect the first freshwater inflow port (210) and the first flow path (241), and
the first freshwater inflow portion (250) is provided to achieve heat exchange between the second surface (12) of the solar cell panel (10) and the influent first freshwater,
wherein the first freshwater inflow portion (250) has at least one surface forming a space into which the first freshwater flows, the surface being the second surface (12) of the solar cell panel (10), and
wherein when the first freshwater flows into the first freshwater inflow portion (250), the first freshwater is brought into contact with the second surface (12) of the solar cell panel (10).

2. The salinity gradient and solar energy hybrid power generation apparatus of claim 1, further comprising a desalination unit (150) supplied with electrical energy produced by the salinity gradient power generation unit (130).

3. The salinity gradient and solar energy hybrid power generation apparatus of claim 2, wherein,
the salinity gradient and solar energy hybrid power generation apparatus (110) is disposed at a front or rear stage of the desalination unit (150),
second saltwater having a lower concentration than the first saltwater flowing out of the salinity gradient and solar energy hybrid power generation apparatus (110) is supplied to and then desalinated by the desalination unit (150) when the salinity gradient and solar energy hybrid power generation apparatus (110) is disposed at the front stage of the desalination unit (150), and
the first saltwater supplied to the salinity gradient power generation unit (130) is discharged from the desalination unit (150) when the salinity gradient and solar energy hybrid power generation apparatus (110) is disposed at the rear stage of the desalination unit (150).

4. The salinity gradient and solar energy hybrid power generation apparatus of claim 1, further comprising at least one ion filter (3121, 3122, 3123, 3124) provided to filter out organic matter or a polyvalent ion contained in the supplied first saltwater and first freshwater.

5. The salinity gradient and solar energy hybrid power generation apparatus of claim 1, further comprising a pre-treatment unit (145,146) provided to remove a floating substance contained in the first freshwater and the first saltwater before the first freshwater and the first saltwater are supplied to the solar photovoltaic power generation and/or solar thermal power generation unit (120) and the salinity gradient power generation unit (130), respectively.

6. The salinity gradient and solar energy hybrid power generation apparatus of claim 1, wherein the salinity gradient power generation unit (130) further comprises a first inflow member (260) provided to distribute the first freshwater to flow into a plurality of the first flow paths (241) without flowing into the second flow path (242).

7. The salinity gradient and solar energy hybrid power generation apparatus of claim 6, wherein the first freshwater inflow portion (250) further comprises a first connection flow path (280) configured to fluidly connect the first freshwater inflow portion (250) and the first inflow member (260) to deliver the first freshwater toward the first flow path (241).

8. The salinity gradient and solar energy hybrid power generation apparatus of claim 1, wherein the plurality of ion exchange membranes (240) comprise a cation exchange membrane (C) and an anion exchange membrane (A), and the cation exchange membrane (C) and the anion exchange membrane(A) are alternately placed.

9. The salinity gradient and solar energy hybrid power generation apparatus of claim 1, further comprising a marine microalgae culture device (2150) supplied with effluent water of the salinity gradient and solar energy hybrid power generation apparatus (110).

10. The salinity gradient and solar energy hybrid power generation apparatus of claim 2, wherein the desalination unit (150) is a combination of a reverse osmosis (RO) apparatus and a pressure retarded osmosis (PRO) apparatus.

## Patentansprüche

1. Eine hybride Salinitätsgradienten- und Solarenergie-Stromerzeugungsvorrichtung, die Folgendes beinhaltet:
eine solarphotovoltaische Stromerzeugungs- und/oder solarthermische Stromerzeugungseinheit (120), der erstes Süßwasser als Kühlwasser zugeführt wird, die ein Solarzellenpanel (10) aufweist, das eine erste Oberfläche (11) mit einem Lichtempfangsabschnitt und eine der ersten Oberfläche gegenüberliegende zweite Oberfläche (12) aufweist, und
eine Salinitätsgradienten-Stromerzeugungseinheit (130), der erstes Salzwasser und das erste Süßwasser, dessen Temperatur aufgrund Wärmeaustausch mit dem Solarzellenpanel (10) erhöht ist, zugeführt wird, um Elektrizität durch einen Salinitätsgradienten zwischen dem ersten Süßwasser und dem ersten Salzwasser zu erzeugen, wobei die Salinitätsgradienten-Stromerzeugungseinheit (130) Folgendes beinhaltet:
ein Gehäuse (200) mit einer ersten Süßwasser-Zulauföffnung (210), einer ersten Süßwasser-Ablauföffnung (211), einer ersten Salzwasser-Zulauföffnung (212) und einer ersten Salzwasser-Ablauföffnung (213);
eine Anode (220) und eine Kathode (230), die in dem Gehäuse (200) angeordnet sind und mit einem vorgegebenen Abstand zueinander beabstandet sind; und
eine Vielzahl von lonenaustauschmembranen (240), die zwischen der Anode (220) und der Kathode (230) vorgesehen sind, um einen ersten Strömungsweg (241), durch den das erste Süßwasser fließt, und einen zweiten Strömungsweg (242), durch den das erste Salzwasser fließt, abzuteilen,
wobei das Gehäuse (200) einen ersten Süßwasser-Zulaufabschnitt (250) aufweist, der konfiguriert ist, um die erste Süßwasser-Zulauföffnung (210) und den ersten Strömungsweg (241) fluidmäßig zu verbinden, und
wobei der erste Süßwasser-Zulaufabschnitt (250) bereitgestellt ist, um einen Wärmeaustausch zwischen der zweiten Oberfläche (12) des Solarzellenpanels (10) und dem zulaufenden ersten Süßwasser zu erreichen,
wobei der erste Süßwasser-Zulaufabschnitt (250) mindestens eine Oberfläche aufweist, die einen Raum bildet, in den das erste Süßwasser fließt, wobei die Oberfläche die zweite Oberfläche (12) des Solarzellenpanels (10) ist, und
wobei das erste Süßwasser, wenn es in den ersten Süßwasser-Zulaufabschnitt (250) fließt, mit der zweiten Oberfläche (12) des Solarzellenpanels (10) in Kontakt gebracht wird.

2. Hybride Salinitätsgradienten- und Solarenergie-Stromerzeugungsvorrichtung gemäß Anspruch 1, die ferner eine Entsalzungseinheit (150) beinhaltet, der elektrische Energie zugeführt wird, die von der Salinitätsgradienten-Stromerzeugungseinheit (130) erzeugt wird.

3. Hybride Salinitätsgradienten- und Solarenergie-Stromerzeugungsvorrichtung gemäß Anspruch 2, wobei
die hybride Salinitätsgradienten- und Solarenergie-Stromerzeugungsvorrichtung (110) an einer vorderen oder hinteren Stufe der Entsalzungseinheit (150) angeordnet ist, zweites Salzwasser, das eine niedrigere Konzentration als das erste Salzwasser aufweist und aus der hybriden Salinitätsgradienten- und Solarenergie-Stromerzeugungsvorrichtung (110) herausfließt, der Entsalzungseinheit (150) zugeführt und dann von dieser entsalzt wird, wenn die hybride Salinitätsgradienten- und Solarenergie-Stromerzeugungsvorrichtung (110) an der vorderen Stufe der Entsalzungseinheit (150) angeordnet ist, und
das erste Salzwasser, das der Salinitätsgradienten-Stromerzeugungseinheit (130) zugeführt wird, aus der Entsalzungseinheit (150) abgeleitet wird, wenn die hybride Salinitätsgradienten- und Solarenergie-Stromerzeugungsvorrichtung (110) an der hinteren Stufe der Entsalzungseinheit (150) angeordnet ist.

4. Hybride Salinitätsgradienten- und Solarenergie-Stromerzeugungsvorrichtung gemäß Anspruch 1, die ferner mindestens einen lonenfilter (3121, 3122, 3123, 3124) beinhaltet, der bereitgestellt ist, um organische Stoffe oder ein mehrwertiges Ion, die in dem zugeführten ersten Salzwasser und ersten Süßwasser enthalten sind, herauszufiltern.

5. Hybride Salinitätsgradienten- und Solarenergie-Stromerzeugungsvorrichtung gemäß Anspruch 1, die ferner eine Vorbehandlungseinheit (145, 146) beinhaltet, die bereitgestellt ist, um eine schwimmende Substanz, die in dem ersten Süßwasser und dem ersten Salzwasser enthalten ist, zu entfernen, bevor das erste Süßwasser und das erste Salzwasser der solarphotovoltaischen Stromerzeugungs- und/oder solarthermischen Stromerzeugungseinheit (120) bzw. der Salinitätsgradienten-Stromerzeugungseinheit (130) zugeführt werden.

6. Hybride Salinitätsgradienten- und Solarenergie-Stromerzeugungsvorrichtung gemäß Anspruch 1, wobei die Salinitätsgradienten-Stromerzeugungseinheit (130) ferner ein erstes Zulaufelement (260) beinhaltet, das bereitgestellt ist, um das erste Süßwasser so zu verteilen, dass es in eine Vielzahl der ersten Strömungswege (241) fließt, ohne in den zweiten Strömungsweg (242) zu fließen.

7. Hybride Salinitätsgradienten- und Solarenergie-Stromerzeugungsvorrichtung gemäß Anspruch 6, wobei der erste Süßwasser-Zulaufabschnitt (250) ferner einen ersten Verbindungsströmungsweg (280) beinhaltet, der konfiguriert ist, um den ersten Süßwasser-Zulaufabschnitt (250) und das erste Zulaufelement (260) fluidmäßig zu verbinden, um das erste Süßwasser an den ersten Strömungsweg (241) zu liefern.

8. Hybride Salinitätsgradienten- und Solarenergie-Stromerzeugungsvorrichtung gemäß Anspruch 1, wobei die Vielzahl von lonenaustauschmembranen (240) eine Kationenaustauschmembran (C) und eine Anionenaustauschmembran (A) beinhalten und die Kationenaustauschmembran (C) und die Anionenaustauschmembran (A) abwechselnd platziert sind.

9. Hybride Salinitätsgradienten- und Solarenergie-Stromerzeugungsvorrichtung gemäß Anspruch 1, die ferner eine Meeresmikroalgenkultureinrichtung (2150) beinhaltet, der Abflusswasser der hybriden Salinitätsgradienten- und Solarenergie-Stromerzeugungsvorrichtung (110) zugeführt wird.

10. Hybride Salinitätsgradienten- und Solarenergie-Stromerzeugungsvorrichtung gemäß Anspruch 2, wobei die Entsalzungseinheit (150) eine Kombination aus einer Umkehrosmosevorrichtung (RO(Reverse Osmosis)-Vorrichtung) und einer druckverzögerten Osmosevorrichtung (PRO(Pressure Retarded Osmosis)-Vorrichtung) ist.

## Revendications

1. Un appareil de production d'électricité hybride par gradient de salinité et énergie solaire comprenant :
une unité de production d'électricité photovoltaïque solaire et/ou de production d'électricité thermique solaire (120), à laquelle est fournie de la première eau douce comme eau de refroidissement, qui comporte un panneau de cellules solaires (10) présentant une première surface (11) comportant une portion réceptrice de lumière et une deuxième surface (12) opposée à la première surface, et
une unité de production d'électricité par gradient de salinité (130) à laquelle est fournie de la première eau salée et la première eau douce rehaussée en température en raison d'un échange de chaleur avec le panneau de cellules solaires (10) pour produire de l'électricité par un gradient de salinité entre la première eau douce et la première eau salée, l'unité de production d'électricité par gradient de salinité (130) comprenant :
une boîte (200) comportant un premier orifice d'entrée d'eau douce (210), un premier orifice de sortie d'eau douce (211), un premier orifice d'entrée d'eau salée (212), et un premier orifice de sortie d'eau salée (213) ;
une anode (220) et une cathode (230) disposées dans la boîte (200) et espacées l'une de l'autre par une distance prédéterminée ; et
une pluralité de membranes échangeuses d'ions (240) agencées entre l'anode (220) et la cathode (230) pour compartimenter un premier chemin d'écoulement (241) par lequel s'écoule la première eau douce et un deuxième chemin d'écoulement (242) par lequel s'écoule la première eau salée,
la boîte (200) comportant une première portion d'entrée d'eau douce (250) configurée pour raccorder fluidiquement le premier orifice d'entrée d'eau douce (210) et le premier chemin d'écoulement (241), et
la première portion d'entrée d'eau douce (250) étant prévue pour réaliser un échange de chaleur entre la deuxième surface (12) du panneau de cellules solaires (10) et la première eau douce affluente,
dans lequel la première portion d'entrée d'eau douce (250) présente au moins une surface formant un espace dans lequel entre la première eau douce, la surface étant la deuxième surface (12) du panneau de cellules solaires (10), et
dans lequel lorsque la première eau douce entre dans la première portion d'entrée d'eau douce (250), la première eau douce est mise en contact avec la deuxième surface (12) du panneau de cellules solaires (10).

2. L'appareil de production d'électricité hybride par gradient de salinité et énergie solaire de la revendication 1, comprenant en outre une unité de dessalement (150) à laquelle est fournie de l'énergie électrique générée par l'unité de production d'électricité par gradient de salinité (130).

3. L'appareil de production d'électricité hybride par gradient de salinité et énergie solaire de la revendication 2,
l'appareil de production d'électricité hybride par gradient de salinité et énergie solaire (110) étant disposé au niveau d'un étage avant ou arrière de l'unité de dessalement (150),
de la deuxième eau salée ayant une plus basse concentration que la première eau salée sortant de l'appareil de production d'électricité hybride par gradient de salinité et énergie solaire (110) étant fournie à l'unité de dessalement (150) puis dessalée par celle-ci lorsque l'appareil de production d'électricité hybride par gradient de salinité et énergie solaire (110) est disposé au niveau de l'étage avant de l'unité de dessalement (150), et
la première eau salée fournie à l'unité de production d'électricité par gradient de salinité (130) étant rejetée par l'unité de dessalement (150) lorsque l'appareil de production d'électricité hybride par gradient de salinité et énergie solaire (110) est disposé au niveau de l'étage arrière de l'unité de dessalement (150).

4. L'appareil de production d'électricité hybride par gradient de salinité et énergie solaire de la revendication 1, comprenant en outre au moins un filtre à ions (3121, 3122, 3123, 3124) prévu pour éliminer par filtration de la matière organique ou un ion polyvalent contenu(e) dans la première eau salée et la première eau douce fournies.

5. L'appareil de production d'électricité hybride par gradient de salinité et énergie solaire de la revendication 1, comprenant en outre une unité de prétraitement (145, 146) prévue pour retirer une substance flottante contenue dans la première eau douce et la première eau salée avant que la première eau douce et la première eau salée soient fournies à l'unité de production d'électricité photovoltaïque solaire et/ou de production d'électricité thermique solaire (120) et à l'unité de production d'électricité par gradient de salinité (130), respectivement.

6. L'appareil de production d'électricité hybride par gradient de salinité et énergie solaire de la revendication 1, dans lequel l'unité de production d'électricité par gradient de salinité (130) comprend en outre un premier organe d'entrée (260) prévu pour distribuer la première eau douce afin qu'elle entre dans une pluralité des premiers chemins d'écoulement (241) sans entrer dans le deuxième chemin d'écoulement (242).

7. L'appareil de production d'électricité hybride par gradient de salinité et énergie solaire de la revendication 6, dans lequel la première portion d'entrée d'eau douce (250) comprend en outre un premier chemin d'écoulement de raccordement (280) configuré pour raccorder fluidiquement la première portion d'entrée d'eau douce (250) et le premier organe d'entrée (260) afin de délivrer la première eau douce vers le premier chemin d'écoulement (241).

8. L'appareil de production d'électricité hybride par gradient de salinité et énergie solaire de la revendication 1, dans lequel la pluralité de membranes échangeuses d'ions (240) comprennent une membrane échangeuse de cations (C) et une membrane échangeuse d'anions (A), et la membrane échangeuse de cations (C) et la membrane échangeuse d'anions (A) sont placées en alternance.

9. L'appareil de production d'électricité hybride par gradient de salinité et énergie solaire de la revendication 1, comprenant en outre un dispositif de culture de microalgues marines (2150) auquel est fournie de l'eau effluente de l'appareil de production d'électricité hybride par gradient de salinité et énergie solaire (110).

10. L'appareil de production d'électricité hybride par gradient de salinité et énergie solaire de la revendication 2, dans lequel l'unité de dessalement (150) est une combinaison d'un appareil d'osmose inverse (RO) et d'un appareil d'osmose à pression retardée (PRO).
